Europäisches Patentamt

European Patent Office

Office européen des brevets

(19)

(11) **EP 0 944 733 B1**

(12) **EUROPEAN PATENT SPECIFICATION**

(45) Date of publication and mention
of the grant of the patent:
**26.03.2003 Bulletin 2003/13**

(21) Application number: **97912418.7**

(22) Date of filing: **18.11.1997**

(51) Int Cl.[7]: **C12Q 1/06**, C12Q 1/18,
G01N 15/14

(86) International application number:
**PCT/IL97/00376**

(87) International publication number:
**WO 98/022618 (28.05.1998 Gazette 1998/21)**

(54) **RAPID MICROBIOLOGICAL ASSAY**

SCHNELLER MIKROBIELLER TEST

PROCEDE RAPIDE DE DOSAGE MICROBIOLOGIQUE

(84) Designated Contracting States:
**DE ES FR GB IT**

(30) Priority: **19.11.1996 IL 11964496**

(43) Date of publication of application:
**29.09.1999 Bulletin 1999/39**

(73) Proprietors:
• **Trek Diagnostic Systems, Ltd.**
**Doar Glil Yam 46905 (IL)**
• **Trek Diagnostic Systems, Inc.**
**West Lake, Ohio 44145 (US)**

(72) Inventors:
• **SAHAR, Eli**
**67895 Tel Aviv (IL)**

• **SCHORR, Amir**
**62917 Tel Aviv (IL)**
• **AHARON, Roni**
**43000 Raanana (IL)**

(74) Representative: **Marchant, James Ian et al**
**Elkington and Fife,**
**Prospect House,**
**8 Pembroke Road**
**Sevenoaks, Kent TN13 1XR (GB)**

(56) References cited:
**WO-A-90/03440          WO-A-93/21511**

• **PATENT ABSTRACTS OF JAPAN vol. 18, no. 571
(C-1267), 2 November 1994 & JP 62 009790 A
(IDEMITSU KOSAN COMPANY LTD)**

EP 0 944 733 B1

## Description

### FIELD OF THE INVENTION

The present invention is in the field of microbiological assays and concerns a method and system for the rapid determination of the presence and level of microorganisms in a liquid sample. Furthermore, the invention also concerns a method and system for rapidly determining the identity of the microorganisms as well as the sensitivity of the microorganisms to anti-microbial drugs. A particular embodiment of the invention concerns the determination of a microorganismal infection in a body fluid.

### PRIOR ART

The following is a list of prior art considered to be relevant as a background to the present invention. The publications will be referred to by their number in the list.

1. Isenberg *et al.*, 1985. In: Manual of Clinical Microbiology, Edwin H. Lennette, Albert Balows, William J. Hausler, J.R., M. Jean Shadomy, (eds.) 4th Ed. *American Society for Microbiology,* Washington, D.C. pp. 73-98.
2. Washington, *et al., Ibid*, pp. 967-987.
3. Pezzlo *et al.*, 1982, *J. Clin. Microbiol.* **15**:468-474.
4. Bates, 1982. *Lab. Manag.*, **20**:7-13.
5. Donta *et al.*, 1981, *N. England J. Med.*, **304**:939-943.
6. Kass, 1978, J. *Infect. Dis.,* **138**:546-557.
7. D'Amato *et al.*, 1985. In: Manual of Clinical Microbiology, Leunette C.H., Balows, A., Hauslower, W.J., Jr., Shadomy, H.J. (eds.) *American Society for Microbiology,* Washington, D.C., pp. 52-65.
8. Thornsberg *et al.*, 1985, *Ibid.*, pp. 1015-1018.
9. Baird-Parker, 1989. In: Rapid Methods and Automation in Microbiology and Immunology, Brixia Academic Press, Brescia, Italy, 276-281, Balows, A., Tilton, R.C., Turano, A. (eds.).
10. Isenberg, 1989, *Ibid.*, 320-326.
11. Vincent *et al.*, 1989, *Ibid.,* 326-332.
12. Sanbolle *et al.*, 1989, *Ibid.*, 333-341.
13. Thabaut, 1989, *Ibid.*, 342-352.
14. Johnston, 1989, *Ibid.*, 353-359.
15. Kelly *et al.*, 1981, *J. Clin. Microbiol.*, **13**:677-680.
16. Hale *et al.*, 1981, *J. Clin. Microbiol.*, **13**:147-150.
17. Aldrige *et al.*, 1977, *J. Clin. Microbiol.*, **6:**406-413.
18. Pfaller, 1985, *J. Clin. Microbiol.*, **21**:783-787.
19. Bixler-Forell *et al.*, 1985, *J. Clin. Microbiol.*, **22**:62-67.
20. Cady *et al.*, 1978, *J. Clin. Microbiol.*, **7**:273-278.
21. Morgan *et al.*, 1983, *J. Clin. Microbiol.*, **18**:384-388.
22. Wu *et al.*, 1981, *J. Clin. Microbiol.*, **21**:796-799.
23. Wallis *et al.*, 1981, *J. Clin. Microbiol.*, **14**:342-346.
24. Perry *et al.*, 1982, *J. Clin. Microbiol.*, **15**:852-854.
25. Marr *et al.*, 1975, *Am. J. Dis. Child.,* **129**:940-943.
26. Atkinson, *et al.*, 1984. In: Anti-microbial Therapy, A.M. Ristuccia and B.A. Cunha (eds.) Rowen Press, New York, pp. 23-36.
27. Greenwood, 1985. In: Rapid Methods and Automation in Microbiology and Immunology, K.O. Habermehc (cds.), Springer Verlag, Berlin, pp. 479-509.
28. PCT Application WO 93/21511.

### BACKGROUND OF THE INVENTION

Microbiological tests have a wide range of applications such as in medicine, in monitoring water quality, in assuring food safety etc. In clinical practice microbiological assays are of particular importance and have a major role in the diagnosis of infections and/or determination of a proper drug treatment in case of infection. In such assays the presence of pathological microorganisms in clinical samples, their number, their identity as well as their sensitivity to antibiotic drugs are determined.

The most widely used microbiological assays involve incubating a biological sample on various growth media and determining the growth of microorganisms thereon[1]. In order to determine sensitivity of the microorganisms to a drug growth of the microorganisms (or lack thereof) is determined in the presence of the tested drug[2]. The main drawback of such assays is that they require considerable time for carrying out, usually 24 to 48 hours, and thus do not enable a rapid diagnosis which is required in cases where immediate treatment is necessary. As a result, very often physicians are compelled to begin treatment without waiting for the clinical results which thus have only a confirmatory value. Consequently, the treatment is not always appropriate and this may at times give rise to severe consequences. Furthermore, the treatment may at times also be given to non-infected individuals. In addition, the fact that the physician has no knowledge on the causative organism, compels him to use broad spectrum antibiotics, which practice results in the development of resistant microorganisms. In addition, the unnecessary administration of antibiotics to patients, is a heavy financial burden on the medical system.

Additionally, such assays are labor consuming and require considerable amount of laboratory space and this considerably limits the widespread use of such assays in screening of susceptible populations for the occurrence of infections. For example, urinary symptoms[3] and elderly people in which asymptomatic infections may be dangerous[4-6], may not be routinely tested.

It may thus be readily understood that methods which do not involve prolonged incubation periods and in which the detection, identification and determination of the sensitivity of microorganisms to antibiotic drugs is rapid, would be highly desirable.

There are a number of rapid methods and systems for detecting microorganisms and determining their sensitivity to antibiotic drugs which are currently available (see for example [7-14]). By one type of such methods and systems the detection of the microorganisms and the determination of their sensitivity to antibiotic drugs, involves a certain initial incubation period of up to about 24 hours. In addition to the time involved, such methods and systems suffer from serious drawbacks in that they give rise to a large proportion of false positive as well as false negative results. Furthermore, these methods and systems arc often not sensitive to concentrations of microorganisms below about $10^4$ or some times even $10^5$ microorganisms/ml. Examples of assays which belong to this group are the Autobac System[15-16], the Auto Microbic System[17-19], the Impedance system[20], and the Bactcc System[21].

Another group of such methods do not require an initial incubation period and therefore are more rapid. However, all these methods have a serious drawback in that they do not enable the determination of sensitivity of the microorganisms to antibiotic drugs and in that, similarly as above, they have a problem of a high rate of false positive and false negative results and they are incapable of detecting microorganisms in concentrations of less than about $10^4$-$10^5$ microorganisms/ml. Examples of such assays are the Bac-t-Screen™[18-23], the Leucocyte Esterase Activity Assay[18,22,24], the Nitrite Test[18,22,25] and the ATP Assay[25,22,26]. A review of rapid automated assays can be found in Greenwood (1985)[27].

International Patent Application, WO 93/21511[28] discloses a method for distinguishing between a microorganism and a non-microorganismal object in a sample based on the spatial distribution of brightness throughout each of the objects viewed in the microscope. A particular application of this method which is disclosed therein is the determination of the sensitivity of the microorganisms to anti-microbial drugs. For such determination, the microorganisms are incubated with the anti-microbial drugs, for a relatively short period of time, and then the sensitivity to a specific drug is determined on the basis of a number of parameters including comparison of the bacterial count following incubation with the drug as compared to control, as well as determination of various morphological changes of the microorganisms following incubation.

## GENERAL DESCRIPTION OF THE INVENTION

The present invention has as its object the provision of a rapid diagnosis assay for determining of the presence and level of microorganisms in a liquid sample and also to determine the type of the microorganisms as well as their sensitivity to anti-microbial agents.

The present invention involves incubating a specimen of the tested liquid sample under a variety of conditions, then filtering the specimen through a filter sheet having pores of a size such that does not allow permeation therethrough of the microorganisms which are thus retained on the filter. Following filtration, the filter sheet is viewed in a microscope which typically has an integral electronic image acquisition device coupled to a computerized image analysis unit. In order to facilitate viewing of the microorganisms, the tested specimens are incubated with dye-comprising reagent systems, the dyes being preferably fluorescent dyes. Where a computerized image analysis method is used for analyzing the microscopic images, a distinction between microorganisms and non-microorganismal objects, as well as between different types of microorganisms and between them and other cellular material, is made by employing an algorithm which can discern between a microorganism and a non-microorganismal object based on a number of morphological parameters. Such an algorithm may, for example, be that disclosed in PCT Application WO 93/21511.

In addition, in order to better distinguish between different kinds of microorganisms, to distinguish between dead and live microorganisms, etc. various staining protocols of which exemplary ones will be described further below, are employed.

**EP 0 944 733 B1**

The present invention provides in accordance with one of its aspects, a method for identifying the type of microorganisms contained in a liquid sample, comprising:

(a) preparing a plurality of specimens from the sample;
(b) obtaining a plurality of microorganism-specific staining agents and adding each such agent to at least one specimen, and then incubating for a time sufficient to allow incorporation or binding of the microorganism specific agent to the microorganisms;
(c) filtering the specimens through a filter having pores of a size such that said microorganisms are retained on said filter;
(d) transferring the filter to a stage of a microscope for viewing by the microscope; and
(e) detecting and optionally counting the stained microorganisms retained on the filter, the presence of stained microorganism indicating the presence of a specific microorganism to which the stain is specific in the sample.

While the enumeration of the microorganisms in step (e) can be performed manually, in accordance with a preferred embodiment of the invention, the microscope forms part of a computerized image analysis system. Typically, such a system comprises an image acquisition unit attached to the microscope which may be a video camera or a CCD device; this image acquisition unit is connected to a computerized image analysis unit, as generally known *per se.*

The sample to be tested may be any liquid sample. In accordance with one preferred embodiment of the invention, the liquid sample is a body fluid and the method then serves as a clinical assay for determination of the occurrence of an infection in the assayed body fluid. A particular example of the body fluid is urine, although other body fluids may also be tested such as blood, lymph, cerebro spinal fluids (CSF), peritoneal fluid (PF), sputum, mouthwash, etc. In addition to body fluids, the assayed liquid sample may be any liquid in which it is desired to determine the presence and concentration of microorganisms, examples being milk, food products, water samples from water reservoirs, and many others.

The present invention also provides, by another of its aspects, a method for evaluating the sensitivity of microorganisms in a liquid sample to an anti-microbial agent, the method comprising:

(a) obtaining data on type of microorganisms in the sample by a method as defined above;
(b) preparing a plurality of liquid specimens from the sample;
(c) adding the anti-microbial agent to at least one specimen and incubating for a time sufficient for the anti-microbial agent to exert an effect on microorganisms sensitive thereto; at least one other specimen serving as control and being incubated under identical conditions without adding an anti-microbial agent;
(d) adding a stain based on said data such that where said data indicates the presence in a sample of a single population of microorganisms, adding a general microorganism stain, and where said data indicates the presence of more than one population of microorganism, adding a general stain, and adding a specific stain for each type of microorganism in the samples, save for one;
(e) filtering the specimens through a filter having pores such that said microorganisms are retained on said filter;
(f) transferring the filter with the retained microorganisms to a stage of a microscope for viewing by the microscope; and
(g) detecting microorganisms on the filter and evaluating their sensitivity to the anti-microbial agent based on a change in one or more properties of the microorganisms, comprising:

(ga) the number of live microorganisms;
(gb) morphological parameters of the microorganisms; and
(gc) biochemical properties manifested by color or color pattern in the microorganisms.

In accordance with a preferred embodiment of the above evaluation method, the microorganisms are evaluated for their sensitivity to a number of anti-microbial agents, wherein in step (c) each specimen except the control specimen is incubated with one of said number of anti-microbial agents.

As can be appreciated, the above methods allow an enumeration, identification and determination of the sensitivity of the microorganism to anti-microbial agents. Thus, for example, the identity of the microorganism present in a liquid sample may first be determined by the above identification method. The decision as to which anti-microbial agents should be tested on the microorganisms present in the sample in the sensitivity test can then be based on the identification results rather then made randomly.

The present invention still further provides, by another of its aspects, a system for carrying out a microbiological assay, useful for the performance of the above methods, the system comprising:

(a) a sample placing station for introduction of samples to be assayed;

(b) one or more devices comprising or capable of holding a plurality of liquid reagent containing vessels;

(c) a device for holding a plurality of specimen plates, each comprising a plurality of specimen holding compartments, and a device for holding a plurality of filter units, each comprising a stretched filter sheets;

(d) a filtration unit comprising a vacuum stage for filtering specimens contained in the specimen plate through the filter sheet of the filter unit;

(e) an incubator unit adapted for holding a plurality of specimen plates;

(f) a microscope unit comprising a microscope, a stage adapted for holding a filter unit for viewing of particulate matter contained on the filter sheet by the microscope, the microscope having an integral image acquisition device connected to a computerized image analysis system; and

(g) a robotic manipulating unit, having grippers for holding and manipulating sample containing vessels, specimen holding plates, and filtering units, and having dispenser devices for collecting liquid samples, specimens and reagents and discharging them into compartments of the specimen plates.

In the following, the invention will be described at times with specific reference to the testing of body fluids, particularly urine. It will be appreciated that this is an example only and the method of the invention may be practiced *mutatis mutandis* on a wide variety of other liquid samples.

## I. Screening of samples (enumeration of microorganisms)

Clinical diagnosis of body fluids, which is a specific embodiment in accordance with the invention, involves at a first stage the screening of body fluids for such, which are classified as infected and to others which are classified as infection free. Such classification depends on a number of factors including:

i. The type of the tested body fluid: Where the body fluid is blood, it has to be totally free of microorganisms and the presence of even few, and at times even a single microorganism, may be hazardous. This is true also for CSF, PF as well as lymph fluid. In the case of urine, mouthwash fluid or sputum, the requirements are not so rigorous, as there is a natural microorganismal flora which exists even in healthy individuals. In such cases, there is usually an acceptable standard, the *"threshold level"*, wherein a level of microorganisms above this level in an individual classifies him as being infected. For example, in a case of *E. coli* in a urine sample, the threshold level commonly used is usually about $10^5$ bacteria/ml and an individual found to have more than this level of microorganisms in his urine is considered as having an infection.

ii. The type of individual: In the case of urine, sputum, mouthwash, etc. the threshold level depends also on the type of individual. For example, in infants as well as in elderly individuals, the threshold level is usually set to be lower than in youth or mid-aged individuals.

iii. The manner of obtaining the clinical sample: In case of a body fluid, the threshold level also depends on the manner of how the clinical sample was obtained from the individual. For example, in case of urine, the results will depend whether the urine sample was a midstream sample (testing of the midstream sample is usually the standard in urine testing) or whether the urine sample was obtained by other means, e.g. by aspiration directly from the bladder. In the latter case, the threshold level will be lower than in the case of a midstream urine sample.

Thus normally, when presented for testing, a body fluid sample will be accompanied by data reflecting the various parameters, and this data can be fed into a computer for determination of the threshold level, which will then be compared to the level of microorganisms determined for the specific individual. This will then allow an accurate classification of the individual as healthy versus infected.

In order to facilitate viewing of the microorganisms, the tested specimen is incubated with dyes which stain the microorganisms. Such stains are generally known *per se* and the artisan will have no difficulties in choosing the right stain for a specific diagnostic assay.

A sample, particularly a clinical body fluid sample, contains both dead and live microorganisms, and both will be viewed in the microscope since in addition to live cells, dead cells also absorb and are thus stained with many dyes. In order to correctly assay the microorganismal load of the sample, it is preferable to subtract the number of dead cells from the total cell count, and for this purpose a reagent system which will allow identification of dead cells should be employed. This may typically be achieved by differential staining. For example, the assayed specimen may be incubated with a reagent system comprising a first dye which stains both live and dead microorganisms and a second dye, having or imparting on cells a different color, which stains only dead microorganisms (i.e. a dye to which the membrane of the live microorganisms is not permeable, but which enters and stains dead microorganisms as a result of their membrane becoming permeable to the dye). Consequently, live microorganisms will be stained only with the first dye whereas the dead microorganisms will become stained with both dyes.

Alternatively, although less preferred, where the two dyes have or impart on the cells, a similar color, two specimens will be incubated simultaneously, one with a reagent system comprising the first dye and another with a reagent system comprising the second dye; the live bacterial count will then be determined by subtracting the dead microorganismal count obtained in one specimen from the total microorganismal count obtained in the other.

The dyes in the reagent system will typically be fluorescent dyes, this in view of the fact that most filter sheets are not fully transparent and therefore it will be difficult to view the cells by bottom illumination. Consequently, the microscope preferably comprises a light system suitable for fluorescent microscopy.

Infectious bacteria, as known in the art, comprise a large variety of microorganisms including Gram negative (Gram⁻) and Gram positive (Gram⁺) bacteria, fungi, particularly yeasts and others. As known, rod-shaped Gram⁺ bacteria such as *lactobacilli, corynebacteria*, etc. are generally considered to be non-pathogenic in urine specimens. Thus, in order to obtain a measure of pathogenic bacterial load in a urine sample, it is necessary to distinguish between the rod-shaped Gram⁺ bacteria and the rest. Accordingly, one or more specimens from the same sample are processed simultaneously and incubated with a reagent system which allows to distinguish between Gram⁺ and Gram⁻ bacteria. For example, a single specimen may be incubated with a reagent system comprising one non-specific dye and another specific for Gram⁺ bacteria, each having or imparting on the cells a different color, and as a result of this differential staining, similarly as above in relation to the dead/live bacteria staining, the Gram⁺ and Gram⁻ bacteria may be distinguished from one another. Again, similarly as above, where the two dyes have a similar color, two specimens may be processed simultaneously and the count for Gram⁻ and Gram⁺ bacteria may be obtained by combining the results of the two specimens. Then, the rod-shaped Gram⁺ bacteria, identified by their morphology, may be counted and this number may then be subtracted from the total live bacterial count in order to obtain a count of pathogenic bacteria. This then gives a more correct measure of the pathogenic load of the urine sample.

As will be appreciated, where the body fluid sample is for example blood or CSF, it has to be totally free of microorganisms and there also Gram⁻ rod-shaped bacteria will be considered as pathogenic. Accordingly, any detection of microorganisms there, including a Gram⁺ rod-shaped bacteria, will classify the sample as being infected.

A dye specific for Gram⁺ bacterial will often also penetrate dead bacteria including dead Gram⁻ bacteria; accordingly, the results from this specimen incubated with a reagent system capable of distinguishing between live and dead bacterial cells is combined with the result obtained with the reagent system for distinguishing between the Gram⁺ and Gram⁻ cells to obtain a more accurate count of live Gram⁺ cells.

It is also possible in accordance with an embodiment of the invention, to incubate the specimen with a reagent system comprising a plurality of dyes, each having a different kind of cell specificity, with each dye having a different color, so as to obtain extensive data with a single specimen.

A major problem associated with microscopic enumeration of microorganisms is to distinguish between microorganisms and non-microorganismal particulate objects. Such objects may be solid particulate matter or in the case of a clinical sample, also body cells. WO 93/21511, discloses a computerized image analysis method which allows to distinguish between microorganisms and non-microorganismal objects. Generally, without going into details in the present writing, this distinction is based on measuring the spatial distribution of brightness throughout each of the objects. Various parameters can be deduced therefrom which serve the basis for a decision whether an object is a microorganism or a non-microorganismal object. This includes the so-called *"optical slope"*, which is a change in brightness across the edge of the object, the so-called *"moment"*, which is a product of the light intensity at each point in the object and its distance from the object's center, as well as the object's length, width, area and its overall shape.

Based on the combination of parameters, microorganisms can be distinguished from non-microorganismal objects. In addition, some of these parameters may serve also to distinguish different types of microorganisms from one another, as well as to classify various non-microorganismal objects. For example, *cocci* bacteria and yeasts are similar in that they are basically circular, and are generally similar in appearance, although they may be distinguished from one another, for example, based on their size (yeasts are larger than *cocci* bacteria).

At times, in order to determine the presence and extent of infection in a clinical body fluid sample, it may be important to determine the level of other non-microorganismal cells present therein including, for example, red blood cells, lymphocytes, epithelial cells, etc. The level of epithelial cells may be important, to determine the quality of a clinical sample: in urine, for example, the number of epithelial cells in a sample is an indication of the sample's quality, i.e., whether it was withdrawn properly from the individual.

Where a liquid sample is found to contain an infectious load of pathogenic microorganisms above a threshold level, it is often also necessary to classify the microorganisms as to their exact type as well as to determine their sensitivity to anti-microbial agents to allow to devise a treatment for the extermination of the microorganisms. This is particularly so in the case of a body fluid sample, wherein such information is required for the physician in order to allow him to devise the proper drug treatment to be administered to the individual so as to treat the microbial infection.

## II. Microorganism identification

In order to identify the type of microorganisms detected in a liquid sample, specimens from the samples are incubated with reagent systems comprising microorganism-specific agents, which can bind to, or which can be incorporated by a specific class of microorganisms. Such agents are either *a priori* bound to a label, or can bind to such label after binding to or being absorbed by the microorganismal cells, which label may be a dye or another coloring agent (e.g. an enzyme yielding a color product). Particular examples of microorganismal-specific agents are monoclonal antibodies. The label may, for example, be a fluorescent dye. The label may be *a priori* bound to the monoclonal antibody or alternatively, the label may be bound to a carrier molecule which then specifically binds to the microorganism-specific antibody. The carrier molecule may, for example, be an antibody which specifically binds to the microorganism-specific antibody; alternatively, the antibody may be conjugated to biotin and the carrier molecule may then be avidin. As will be appreciated by the artisan, these two labeling embodiments are only examples of a myriad of other labeling techniques which will allow to identify binding of the microorganismal specific agent to the microorganismal cells.

Typically, a plurality of specimens from the same sample are separately and simultaneously incubated with general reagent systems, each comprising a different microorganism-specific agent, e.g. a different microorganism-specific monoclonal antibody. The incubation is for a time sufficient to allow staining of the cells or in the case of the monoclonal antibodies, a time sufficient to allow binding of the monoclonal antibodies to the microorganismal cells. In the embodiment where use is made by biotin-carrying monoclonal antibodies, it is preferable to add the avidin-label conjugate only after incubation.

One unique feature of the method of the invention is the fact that the labeling reaction is a homogeneous reaction and there is no need for subsequent washing of the specimens to remove undetected label. This is a result of the fact that following incubation, the liquid sample is filtered to view the microorganisms whereby non-bound dyes or coloring agents are filtered through.

It is at times also possible to reduce the number of simultaneously incubated specimens by using reagent systems comprising a plurality of monoclonal antibodies. each with a different microorganism specificity, and each associated with a dye or coloring agent having or giving rise to a different color.

Following incubation and dying, the microorganisms are filtered through a filter and then the filters with the retained microorganisms are transferred for viewing to a microscope, similarly as above.


### III. Testing for sensitivity to anti-microbial drugs

In order to test the sensitivity of the microorganism identified in a liquid sample to anti-microbial drugs, a plurality of specimens from the samples are incubated simultaneously, one specimen serving as control and the others being incubated simultaneously and under the same conditions, each with a different anti-microbial drug. In case of a body fluid sample, the specimens will typically be incubated in an incubator at 37°C. The incubation time will then usually be about 1-3 hours, preferably about 2 hours.

In order to get optimal results, the specimens are initially diluted so as to have an optimal initial number. The dilution is performed on the basis of the enumeration results obtained above.

Based on the microorganism identification results, there may be basically two situations:

   i. the tested liquid sample contains predominantly a single kind of microorganisms;
   ii. the tested liquid sample contains a mixed flora of two or more kinds of microorganisms.

In the former case, following incubation with the antibiotic drug, the liquid specimen is incubated with a reagent system comprising a dye which stains all microorganisms non specifically. In the latter case, where the liquid sample comprises a mixed flora, the specimen is incubated also with microorganism-specific dyes. While it is possible to add a specific dye for each microorganism, it is usually preferred to add one microorganism-non specific dye, i.e. a dye which stains all microorganisms and one dye which stains only a specific class of microorganisms i.e. a microorganism-specific dye), e.g., microorganism-specific monoclonal antibodies. The total number of microorganism-specific dyes will be one less than the number of the different microorganisms in the specimen. For example, where the mixed flora consists of two Microorganisms, (*"Microorganism A"* and *"Microorganism B"*), one general dye which stains all microorganisms, i.e. both Microorganism A and Microorganism B, will be added and then another dye specific, for examplc, for Microorganism A, (e.g. an anti-microorganism A labelled monoclonal antibody) may also be added. Such a differential staining will allow to distinguish between the different microorganisms' populations and allow specific examination of the anti-microbial agent sensitivity of the different microorganisms in the mixed flora.

The sensitivity of the microorganisms to anti-microbial agent will be determined by a combination of a number of parameters including the extent of proliferation of the microorganism cultured in the presence of the anti-microbial agent as compared to the control specimen (cultured simultaneously without the addition of any anti-microbial agent) as well as a change in the microorganism's morphological or biochemical parameters. This may be performed by a computerized image analysis method such as that disclosed in WO 93/21511.

The manifestation of the anti-microbial drug sensitivity is different for different drugs and for different microorganismal species. For example, incubation of microorganisms with cephalosporin will usually cause elongation thereof while incubation of the same microorganisms with amino-glycosides may cause a shortening and thickening; augmentin, as another example causes the formation of *"blobs"* in the septum of sensitive bacteria. This anti-microbial agent specificity or species specificity should be taken into consideration when evaluating the anti-microbial agent sensitivity.

Microorganisms in culture tend to shed antigens which can then be incorporated non specifically by other microorganisms. This is an important feature which has to be considered in the case of a mixed culture: shed antigens from microorganisms of one species may be adsorbed non specifically by microorganisms of another species and this can then obscure the results. In order to avoid such non specific binding, agents that inhibit non specific absorption of antigens may be added to the medium of each specimen during incubation. An example of such an agent is bovine serum albumen (BSA), Tween™, etc.

The invention will now be further illustrated by reference to a specific embodiment concerned with clinical assay of urine samples to enumerate their microorganismal load, to identify the microorganism detected therein and to test for the sensitivity of the microorganism to anti-microbial drugs.

## BRIEF DESCRIPTION OF THE DRAWINGS

**Fig. 1** shows the prior art method for enumeration of microorganisms in a clinical sample and subsequent identification and assaying for sensitivity to anti-microbial drugs of positive samples;

**Fig. 2** is a schematic overview of the method of the invention for enumeration, microorganism identification and assaying for sensitivity of the microorganisms to anti-microbial agents;

**Fig. 3** is a schematic representation of microorganism enumeration in accordance with the invention;

**Fig. 4** is a schematic illustrative representation of a photomicrograph of specimens processed as shown in Fig. 3, wherein **Fig. 4A** shows results of differential staining for dead and live microorganisms and **Fig. 4B** shows results of differential staining for Gram$^+$ and Gram$^-$ microorganisms;

**Fig. 5** shows real examples of micrographs of specimens processed as described in Example 1:

**Figs. 5A** and **5B** show two views of the same specimen incubated with a reagent mixture (identified as SRM-1 in Example 1) for differential staining of the entire microorganismal population by one stain and dead cells by another stain, wherein Fig. 5A shows the results from the general stain showing all cells in the sample and Fig. 5B shows only the dead cells;

**Figs. 5C** and **5D** show a specimen incubated with a reagent system for differential staining of the entire bacterial population and Gram$^+$ bacteria (identified as SRM-2 in Example 1), wherein Fig. 5C shows the entire microorganismal population and Fig. 5D shows the differently stained Gram$^+$ bacteria;

**Fig. 6** is a schematic representation of the method for identifying microorganisms in a liquid sample utilizing **n** microorganism-specific antibodies, each one specific for a different type of microorganism (this assay tests for the presence of the total of **n** different microorganisms);

**Fig. 7** is a schematic representation of the method for assaying for sensitivity of the microorganisms in a sample to anti-microbial agents (in this case the six different agents, marked as "A$_a$", "A$_f$); and

**Fig. 8** is an isometric view of a device useful in carrying out the method of the invention.

## DESCRIPTION OF SPECIFIC EMBODIMENTS

The invention will now be described with reference to the specific embodiments concerned with the clinical testing of urine samples for the purpose of diagnosing urinary tract infections. As will no doubt be appreciated by the artisan, this is an example only and the invention can be practiced, *mutatis mutandis*, also with other types of biological liquid samples.

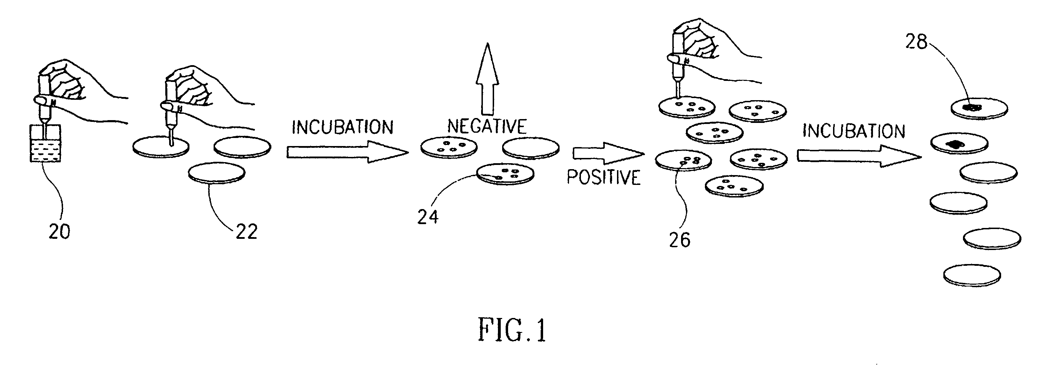

Reference is first being made to Fig. 1, showing the prior art method for screening of urine samples to determine the occurrence of infection and subsequent identification of the type of infectious microorganism and their sensitivity to anti-microbial drugs. The standard prior art method is based on culturing a urine sample on culture plates, incubating the plates for a sufficient amount of time to allow the formation of colonies and then counting the colonies. At a first stage a urine sample **20**, typically a midstream urinary sample is collected and then plated on a number of culture plates **22.** Following incubation, which typically lasts about 24 hours, colonies **26** form on plates **22** which are then viewed and the number of colonies are counted. The number of colonies below a certain critical value is considered a negative result; if the number of colonies exceeds the threshold level, the sample is considered positive, namely, there is an infection. In case of a positive result, the test will typically proceed further in order to determine the type of microorganisms and their sensitivity to anti-microbial drugs.

For this purpose, samples of the developed colonies are taken and plated on a plurality of other culture plates **26.** Some of the plates may have a variety of selective growth media or a variety of color indicators, intended to allow identification of microorganism, based on their pattern of growth on different growth media, and other growth media have added thereto anti-microbial drugs and serve for testing of the microorganisms' sensitivity to such drugs. Following plating, the cultures are typically incubated for a further period of about 24 hours, during which colonies **28** develop on the growth permissible media. The growth of the microorganisms which can again be viewed by means of formation of colonies 28, may serve for characterization of the type of microorganisms as well as for determination of their drug sensitivity.

One drawback of this conventional method is that the results are delayed as they depend on the formation of colonies which typically, in a rapid growing microorganism, is a process which lasts about 24 hours. Thus, it takes about 48 hours to find out the microorganisms' drug sensitivity. At such a late stage, treatment has usually already begun and at times the courses of the disease has already inflicted severe harm to the patient. Furthermore, it should be noted that some microorganisms grow in a much lower rate and considerably longer time is required to obtain results.

Other drawbacks of such a method is that it is labor intensive, requires skilled personnel and furthermore, the manipulation of the plates required at each stage can result in contamination by extraneous sources which may obscure the results.

In addition to the time factor, as noted above, one other problem in such a prior art method arises when an infection is caused by a mixed flora of microorganisms, typically two. In order to test for their sensitivity to anti-microbial drugs, it is necessary to separate between colonies of the different bacteria and test the sensitivity separately for each population. This obviously complicates the procedure. Furthermore, microorganisms in even a single microorganism population may be comprised of different sub-populations of microorganisms differing from one another with respect to their sensitivity to anti-microbial agents. For example, although the population as a whole may be sensitive to a certain drug, a small proportion, e.g. 5-10% may be relatively resistant. In prior art methods, it is usually very difficult, if not impossible to identify such variation in sensitivity as the results obtained are from a single, randomly selected colony. This may result in providing the physician with a recommendation for a certain drug treatment which will not be effective against a certain sub-population of microorganisms which was among those causing the infection.

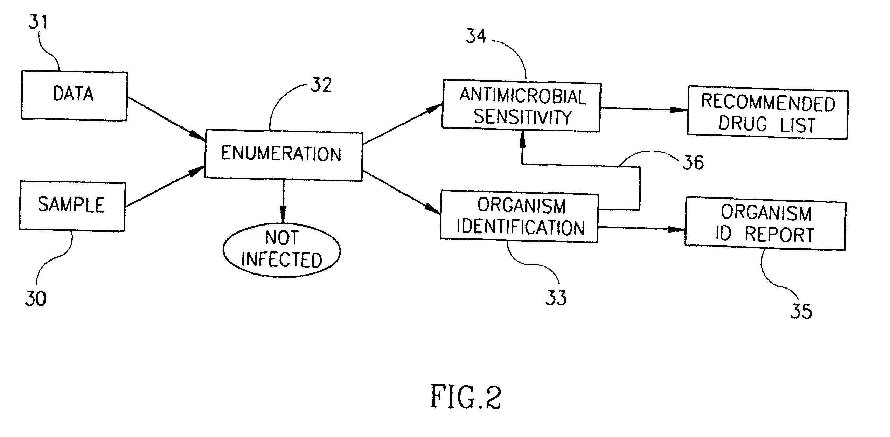

Reference is now being made to Fig. 2, which provides an overall schematic overview of a manner of testing a urine sample for microorganisms enumeration, identification and testing for their sensitivity to anti-microbial drugs. A urine sample **30** is collected, which is preferably a midstream urine sample, and this sample accompanied by data **31** (the manner in which the sample was collected, the type of individual, etc.) is then assayed. The first step **32** is a relatively rapid enumeration step, for screening individuals for such which arc infected and such which are healthy, involving, as will be detailed further below, adding stains and then subjecting a sample, following filtration, to microscopic analysis, which allows enumeration of the number of infectious microorganisms in a sample. In case the result of the enumeration step **32** is negative, i.e. that the number of bacteria is less than a certain critical value, (which critical value depends on the manner in which the sample was collected, the type of individual, etc.) the screened individual is classified as healthy and the test terminates there. The results of this screening step may be available within about 20-30 minutes. In case of a positive result, specimens of the samples are further tested in an assay **33** intended to determine the identity of the infectious microorganisms and another assay **34** intended to determine the microorganisms' sensitivity to anti-microbial drugs. Assays **33** and **34,** are typically performed simultaneously. The identification results **35** are applied also in the anti-microbial agent sensitivity assay **34,** as represented by arrow **36**, in a manner which will be explained further below. Eventually, based on the sensitivity assay, a recommended drug list is provided which allows the physician to design an optimal mode of treatment of the infection. As distinct from the above-mentioned prior art method, results providing such a drug list may be available, within about 3 hours.

Furthermore, in addition to microorganisms, there are also additional indices of infection, such as, for example, the number of polymorphonuclear (PMN) cells, which cannot routinely be determined in the standard diagnosis method. In accordance with the invention, where the method is based on viewing and microscopically measuring a combination of parameters on individual cells, some of these drawbacks are overcome.

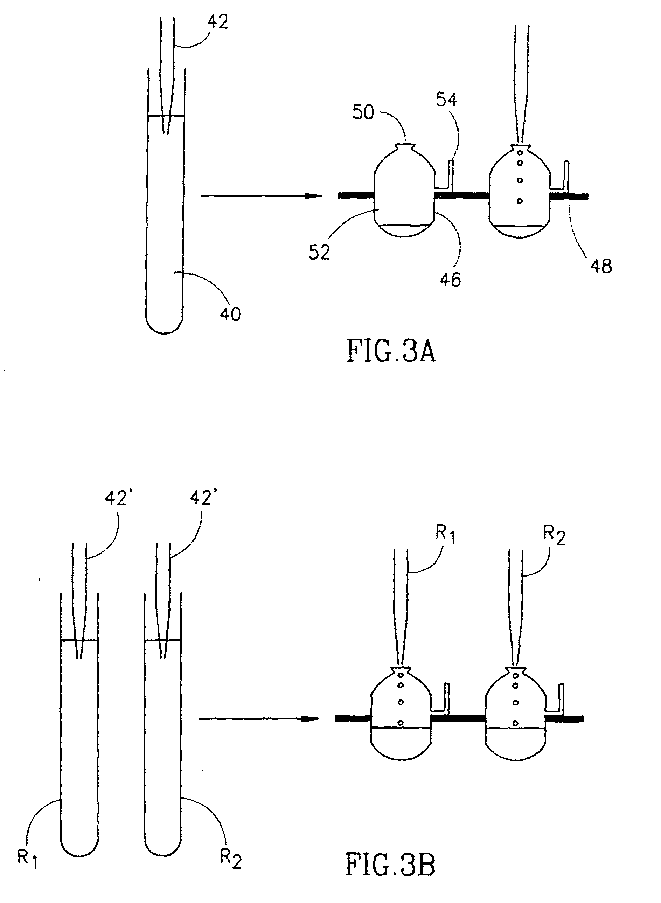

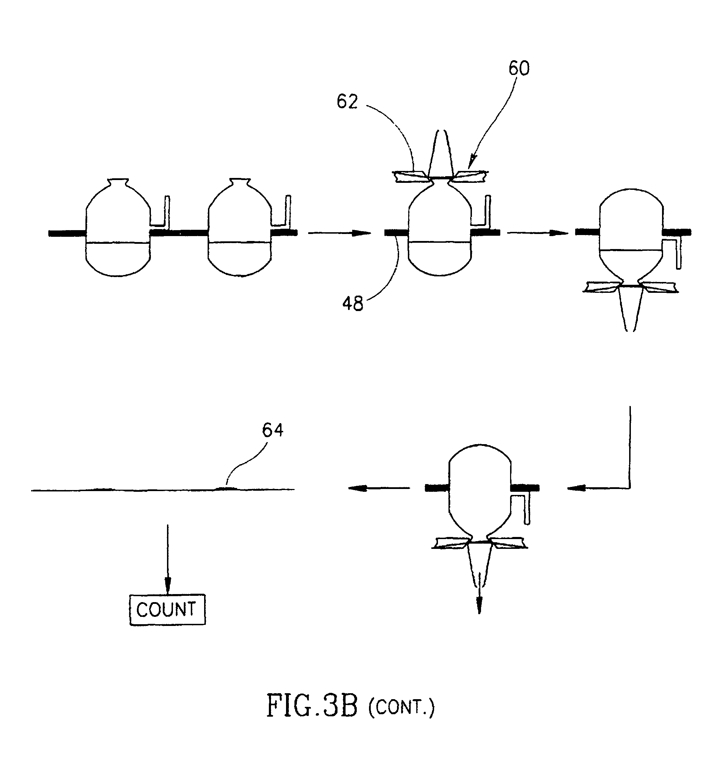

Reference is now being made to Fig. 3, which provides a schematic outline of the method of enumeration of microorganisms (block **32** in Fig. 2) in accordance with the invention. In a first step, shown in Fig. 3A, a plurality of specimens, e.g. each of about 30 μl are collected from a urine sample **40,** e.g. by disposable microtips **42** attached to a dispenser unit of a robotic manipulator system (not shown), and then transferred each to compartments **46** of a multi-compartmental plate **48**. The plate may, for example, be the multi-compartmental plate such as that disclosed in WO 97/41955. Briefly, without going into detail, each compartment **46** in plate **48** has an opening **50,** a liquid incubation space **52** and a nozzle **54** which extends from a side wall of the compartment to the upper face **56** of the plate. This structure of the compartment allows both incubation of a liquid sample and subsequent filtration through a filter sheet (see WO 97/41955). Typically two specimens are collected from sample **40** and transferred each to a different compartment **46.**

At a next stage, shown in Fig. 3B, two different liquid reagent systems, $R_1$ and $R_2$ are collected, again by the use of disposable microtips **42',** and each of these reagent systems $R_1$ and $R_2$ are then dispensed into a different compartment **46.** Reagent system $R_1$ comprises two dyes, one of which stains all cells in the specimen and the other which stains only dead cells. Reagent $R_2$ comprises one dye which stains all cells (which may be the same dye as the first dye in reagent system $R_1$) and a second dye which stains only Gram$^+$ bacteria (It should be noted that some Gram$^+$ dyes sometimes stain also dead cells).

Following incubation, which is typically for about 20 minutes at 37°C, a filter unit **60** comprising a filter sheet **62** is placed on top of plate **48,** as can be seen in Fig. 3C, and the assembly comprising plate **48** and filter unit **60** is then inverted and subjected to a vacuum filtration whereby the liquid phase filters through filter **62** and particulate matter **64** is retained on filter **62.** Filter unit **60** with the filter **62** is then transferred to a microscope where the particulate matter is viewed and the microorganisms are counted. The microscope has typically an integral image acquisition unit, coupled to a computerized image analysis system for analyzing the image and obtaining a microorganismal count.

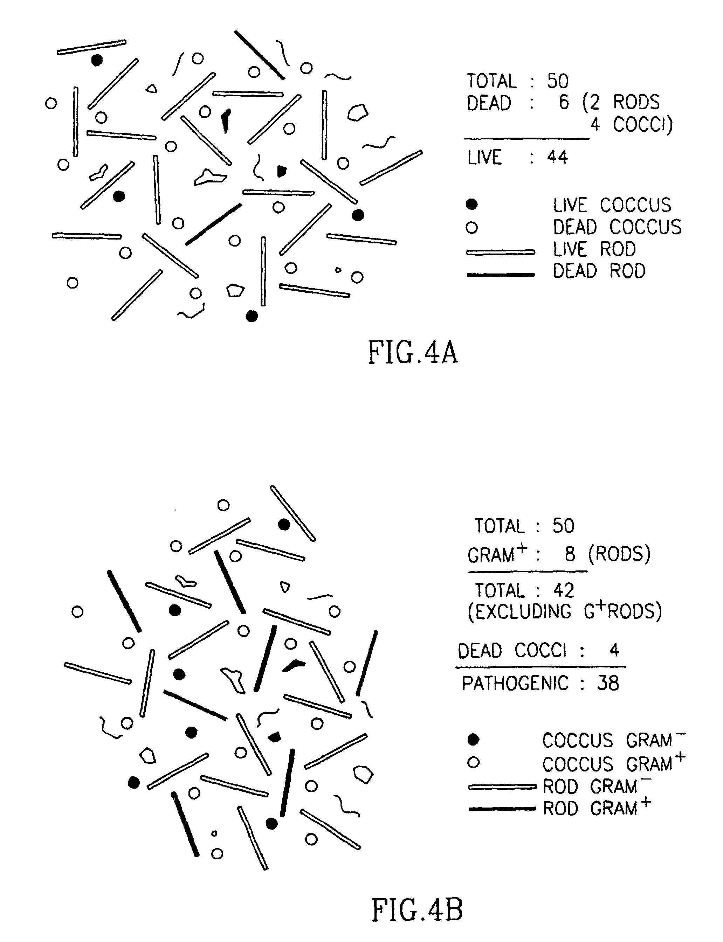

Reference is now being made to Fig. 4 for the purpose of explaining how a microorganismal count is obtained. A clinical sample, typically comprises both dead and live microorganisms as well as microorganisms which are non-pathogenic. As is generally known, Gram$^-$, rod-like bacteria in urine are typically considered to be non-pathogenic microorganisms. In prior art culturing methods it was usually difficult to distinguish non-pathogenic bacteria from pathogenic ones as non-pathogenic bacteria are also capable of growing in culture media and will thus be eventually counted. Thus, in prior art methods, this fact may have led at times to a false positive result.

Fig. 4A is a sketch representing one microscopic field of a specimen obtained following incubation with reagent system $R_1$. This reagent system, as stated above, comprises a first dye which stains all cells (both dead and live cells) and another dye which stains only dead cells. The dyes will typically be fluorescent dyes and the microscope is accordingly adapted for fluorescent illumination. (While it is in principle possible also to use non-fluorescent dyes, fluorescent dyes are preferred seeing that the filter sheet is usually not transparent enough to allow bottom illumination). As can be seen, the specimen comprises both rod-like bacteria and *cocci* (spherical) bacteria. In addition, the specimen comprises also some irregularly shaped, non-cellular material. All bacteria are stained by the first fluorescent dye and this feat allows to count all cells in the micrograph. As can be seen, some of the cells (6 in the specific example, consisting of 2 rod-like and 2 *cocci* bacteria) are darkened, this meaning to signify that they are stained with the dye specific for dead cells. The total number of bacteria in this representation is 50, and deducting therefrom the 6 dead cells, gives a total count of 44 live microorganismal cells. This then allows to calculate the concentration of live cells in the sample.

Out of the live cells, some are pathogenic, and some, which are Gram$^+$ - rod-like shaped bacteria, are non-pathogenic, and thus in order to obtain a better estimate of the infectious load of the sample, it is necessary to deduct from the total number, the number of such non-pathogenic bacteria. This is performed by evaluating the specimen incubated with a reagent system $R_2$. A sketch of a micrograph of such a urine specimen is shown in Fig. 4B. Here again, the total number of bacterial cells which are shown is 50, out of these 12 are darker, representing microorganisms stained by the Gram$^+$ dye (Gram$^+$ bacteria). Out of these, 8 are rods, and arc thus non-pathogenic bacteria. The darkly stained microorganisms include dead microorganismal cells as well as Gram$^+$ rod-like and *cocci* bacteria. Thus, all the lightly stained microorganisms are pathogenic as they are all Gram$^-$ bacteria (which are pathogenic). All the dark stained and rod-like bacteria are non-pathogenic as they consist of either Gram$^+$ or dead cells. Against this, some of the dark stained, *Cocci* bacteria are pathogenic and some are dead cells. The number of dead *Cocci* may then be calculated from a micrograph of the type obtained in Fig. 4A (4 in the specific example) which gives a total count of pathogenic microorganisms of **38.**

Obviously, the numbers are not as represented here but are rather concentration of bacteria in the original sample. Typically, a plurality of fields for each specimen will be tested to obtain a more accurate average result.

The method of enumeration as outlined above, utilized in order to obtain a count of pathogenic live cells, can best be understood by the aid of the following formulae (I) and (II):

The specimen incubated with the $R_1$ reagent system, in the present example where the sample consists only of bacteria, may allow to discern between 4 groups of bacteria:

| | |
|---|---|
| Live *Cocci* | $L_C$ |
| Live Rods | $L_R$ |
| Dead *Cocci* | $D_C$ |
| Dead Rods | $D_R$ |

The number of live bacterial cells may then be calculated by the following formula (I):

$$\text{Live Cells} = L_C + L_R \tag{I}$$

Obviously, where the specimen includes other types of microorganisms, e.g. yeast, these will have to he added as well.

The specimen incubated with the $R_2$ reagent system, allows to discern between 4 groups of bacteria:

| | |
|---|---|
| Gram⁻ Rods | $G_R$ |
| Gram⁻ *Cocci* (usually very rare in urine) | $G_C$ |
| Stained *Cocci* (both Gram⁺ and dead cells) | $S_C$ |
| Stained Rods (both Gram⁺ and dead) | $S_R$ |

The number of pathogenic live cells may then be calculated by the following formula (II):

$$\text{Pathogenic Live Cells} = G_R + G_C + S_C - D_C \tag{II}$$

In the above example, the specimen is shown as consisting only of bacteria (both Rod-like and *Cocci*). A clinical sample will very often contain other cells such as epithelium cells, PMN cells, other blood cells, etc. which may also be identified and separately enumerated.

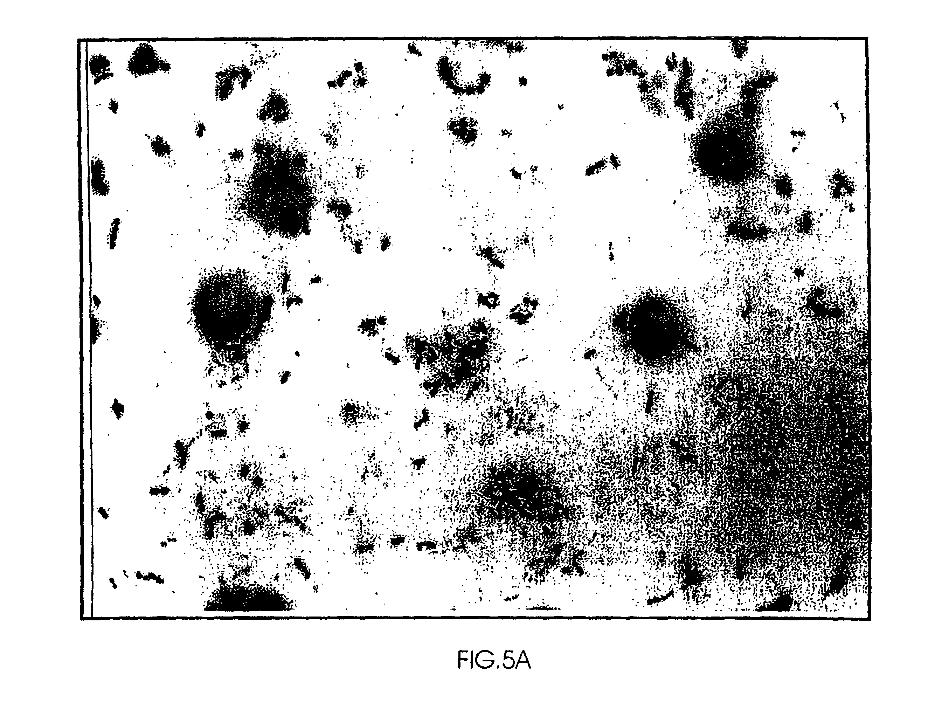

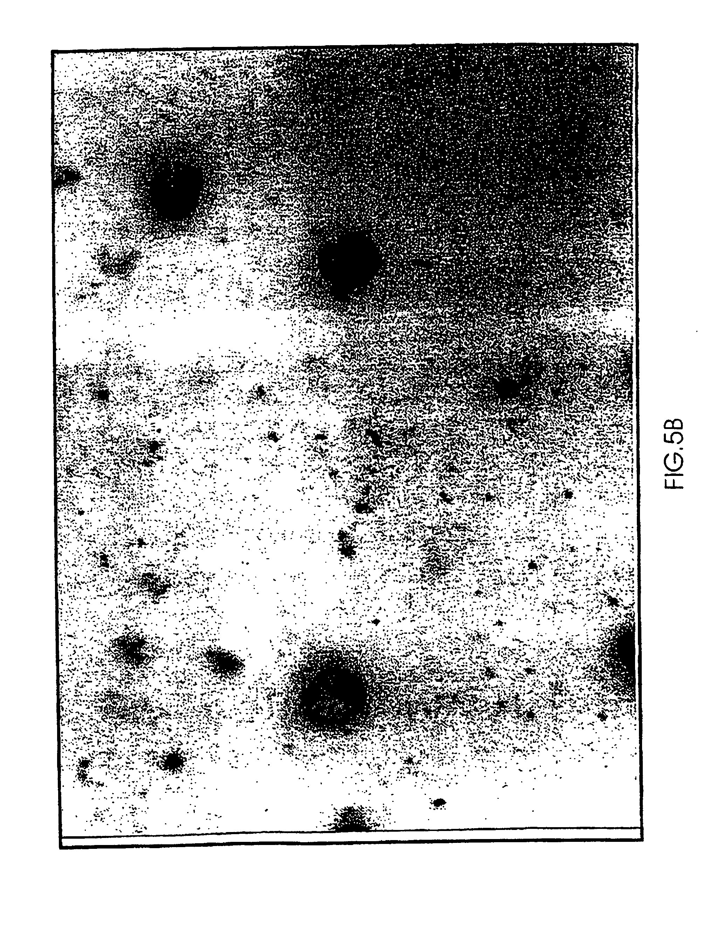

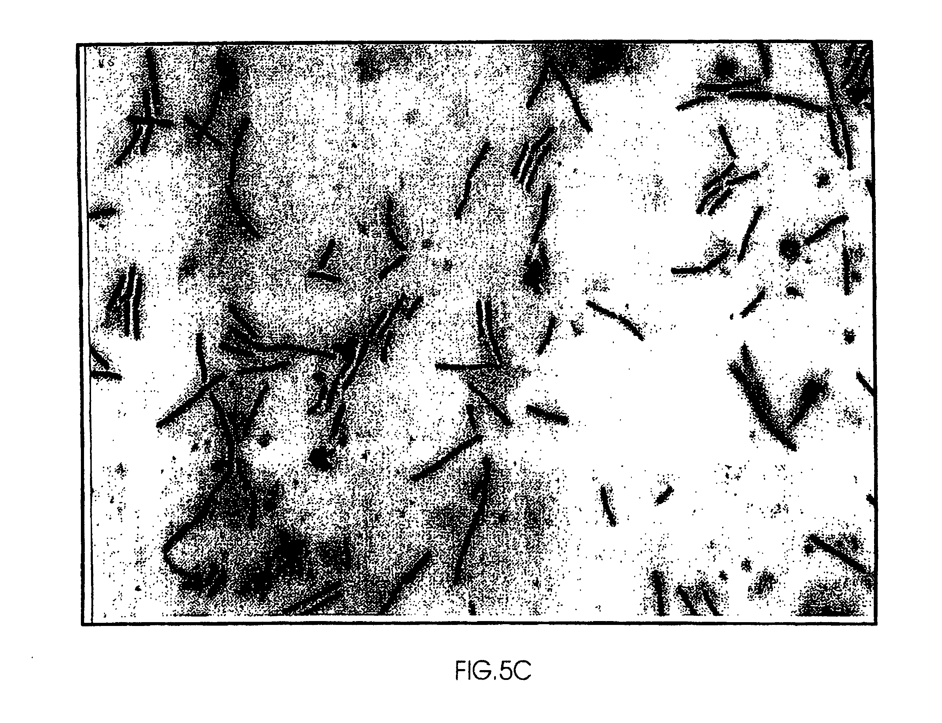

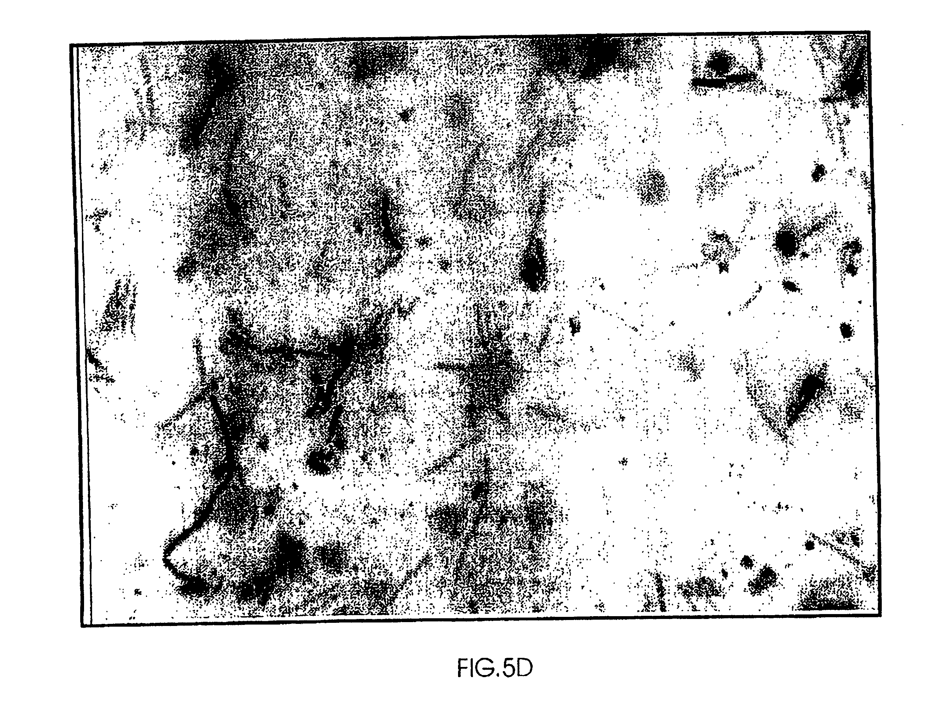

Reference is now being made to Fig. 5 showing an actual example of specimens stained as above. Figs. 5A and 5B show a specimen stained with a general stain to obtain a general bacterial account, and another stain which stains only dead cells. This allows to differentiate between live and dead cells and the computer processed image of the specimen is shown in Figs. 5A and 5B, wherein Fig. 5A shows all cells and Fig. 5B shows only the dead cells. (In actuality the dead cells can be distinguished from the live cells by virtue of the fact that they have a different color; in order to facilitate viewing of these cells it is possible also to use appropriate filters).

Reference is now being made to Figs. 5C and 5D, when Fig. 5C shows the entire cell population, all of which arc stained by a general microorganismal stain, wherein Fig. 5D the Gram⁺ bacteria can be seen (stained by a Gram⁺-specific stain).

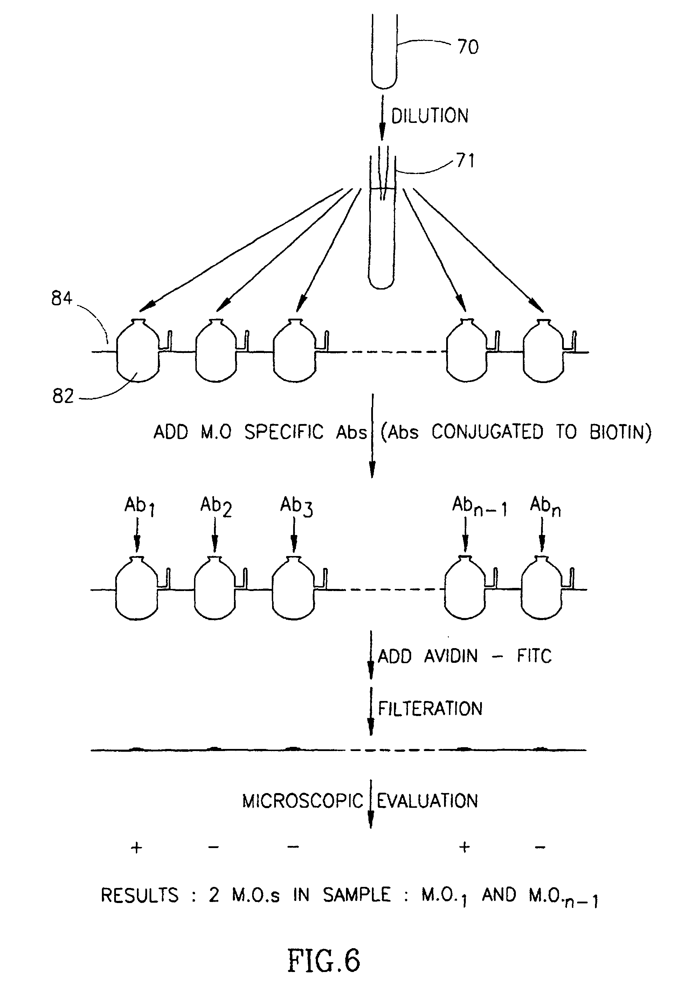

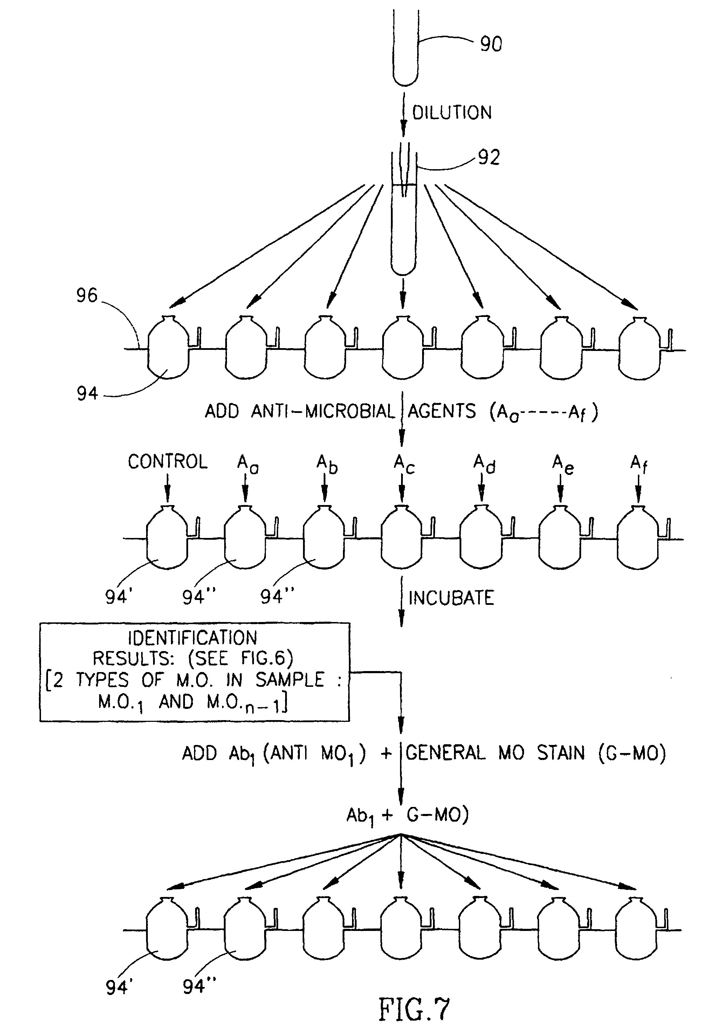

Following a positive result of infection, the sample is then processed further in order to first identify the type of microorganisms and then determine their sensitivity to anti-microbial drugs (blocks **33** and **34** in Fig. 2). Reference is first being made to Fig. 6 which is a schematic representation of the method for identification of the type of microorganisms in the sample. Following the enumeration step, a count of the microorganismal concentration in the original sample is obtained. For the identification as well as for the assaying for the sensitivity of the microorganisms to anti-microbial drugs, it is preferable to start with a certain optimal concentration of microorganisms in a sample, e.g. about $10^5$ cells/ml. For this purpose, the original sample **70** is first diluted to yield a diluted sample **71**, the degree of dilution depending on the enumeration results. A plurality of specimens, e.g. each of about 30 μl, are then dispensed each into one of a plurality of compartments **82** of multi-compartmental plate **84** (plate **84** may be the same as plate **48** of Fig. 3). To each compartment there is then dispensed one of a plurality of monoclonal antibodies, $\textbf{Ab}_1...\textbf{Ab}_n$ which arc each specific for one class of microorganisms. For example, almost all microorganisms which are found in urine belong to one of about 15 species and in order to identify whether the microorganisms in a sample belong to one of those species, a corresponding 15 different monoclonal antibodies are then added each into one of the compartments (in this case n = 15). The monoclonal antibodies may be originally conjugated with a marker, e.g. a fluorescent dye, but preferably, the antibodies are conjugated to a member of a binding couple, e.g. biotin, and can then specifically bind to another member of the couple, e.g. avidin.

The plate with the specimen is then incubated for a time sufficient to allow the specific binding of the monoclonal antibodies to the microorganisms, e.g. about 30-60 minutes at 37°C, following which, if the antibodies are not initially labelled, the labeling complex, for example, avidin, conjugated to a fluorescent tag, e.g. fluorescein-iso-thio-cyanatc (FITC) is then added. Following a short period of incubation of several minutes, the sample is filtrated whereby antibodies not bound to microorganisms as well as the fluorescent marker complex, are filtered through the membranes, with the microorganisms being retained on filter **86**. (The filter may, here again, form part of a filtering unit similarly as in Fig. 3). The filter is then subjected to a microscopic analysis, similarly as that performed for the enumeration (Fig. 3) to identify stained microorganisms. The identification of stained microorganisms in a specimen then signifies the existence of these microorganisms in the original sample. Fig. 5 illustrates the obtaining of positive results for $\textbf{Ab}_1$ and $\textbf{Ab}_n\textbf{-1,}$ which means that the corresponding microorganisms exists in the original sample, identified here as $\textbf{M.0.}_1$ and $\textbf{M.0.}_{n-1}$. (For example $\textbf{M.0.}_1$ may be *E. coli* and $\textbf{M.0.}_{n-1}$ may be *Staphylococcus*).

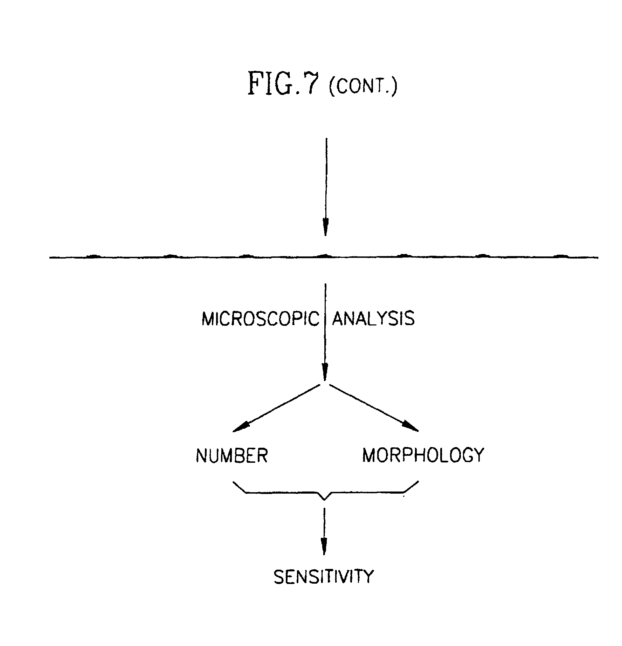

Reference is now being made to Fig. 7 which illustrates, again schematically, the method for determining the sensitivity of the microorganisms to anti-microbial agents. In this specific example, the sensitivity is tested against a panel of such agents (6 in the specific example) marked **$A_a$-$A_f$**.

At a first step, the original sample **90** is diluted to yield a diluted sample **92** from which specimens are then dispensed into seven compartments **94** of a multi-compartmental plate **96.** (Plate **96** may again be similar to plate **48** in Fig. 3). The first compartment, **94'** serves as control and to this compartment a controlled medium which does not contain any anti-microbial drug is added. To each of the other six compartments **94",** a medium containing each one of the six tested anti-microbial agents, **$A_a$-$A_f$** is added.

The multi-compartmental plate with the specimens is then incubated, e.g. for about 90-120 mins. at 37°C. At this time results from the identification procedure (obtained as shown in Fig. 4), which was begun simultaneously with specimens obtained from the same diluted sample, are obtained. These results provide, *inter alia*, information on whether the sample contained predominantly a single population of microorganisms or whether a mixed population comprising more than one type of microorganism. In this specific illustrative example, as already pointed out above, the results indicate the presence of a mixed culture comprising the microorganism **$M.0_1$** and microorganism **$M.0_{n-1}$.**

In case the results show that the sample comprises a population of a single species of microorganisms, a reagent system comprises a stain which generally stains all microorganisms is dispensed in each of the compartments. Where, however, as is the case in this example, the sample is found to contain a mixed population of microorganisms, both a general stain **(G-MO)** and a species-specific stain, e.g. a monoclonal antibody, is added into each of the compartments **94'** and **94".** Where the sample contains more than two species of microorganisms, additional microorganism-specific stains will be added, the total of microorganism-specific agents being one less than the total number of microorganisms in the sample (e.g. in the case of three microorganisms, there will be added two microorganism-specific stains and one general stain).

At the next stage, following incubation for a time sufficient for the microorganisms to become stained, the specimens are filtered through filter **98,** similarly as the filtration described in Figs. 3 and 6, and the particulate matter contained on the filter, which comprises the stained microorganisms is then subjected to microscopic analysis.

The mixture of reagents will allow to identify and classify individually each population of microorganisms. In this specific example, both **$MO_1$** and **$MO_{n-1}$** which exist in the sample, will be stained by the general stain, **G-MO** whereas microorganismal population **$MO_1$** will be stained also by the specific microorganismal stain. Thus, microorganisms which will have both stains will belong to one class and the microorganisms which will show only one stain will belong to another class. Similarly, it is possible also to identify in this manner more than two classes of microorganisms. It will be appreciated, however, that for such differential staining it is necessary that each staining agent will be different than the general stain thus imparting a different color to the microorganisms. Typically, 4-5 different reagents may be simultaneously used.

The micrograph from each specimen is then analyzed and compared to the control. One parameter which is analyzed is a change in bacterial count following incubation. In many cases, exposure of the microorganisms to an anti-microbial drug to which these microorganisms are sensitive, will reduce or arrest their proliferation. Thus, a difference in the number of microorganisms of a specific population as compared to control will indicate sensitivity. However, this may not be sufficient for a number of reasons. For example, the effect of the anti-microbial agent may be time dependent and may exert an appreciable effect only at a later stage. In addition, a microorganismal cell culture may contain sub-populations which have a different sensitivity to a specific anti-microbial agent as compared to the major population, as well as change in biochemical properties reflected by changes in color and staining patterns. Thus, assaying for the sensitivity of the microorganisms to a specific anti-microbial agent also takes into consideration various morphological parameters such as length, width, and overall shape. This is typically performed by a computerized analysis system such as that disclosed in WO 93/21511. Generally, without going into details here, the various parameters are given a score, e.g. 0.5 to the degree of proliferation and 0.5, equally divided, to the degree of change in morphological parameters, and a change in this score is then compared to control and allows an evaluation of sensitivity.

The score given to each parameter is to some extent empiric and depends on the type of anti-microbial agent, on the specific species of microorganism concerned, and may be adjusted accordingly.

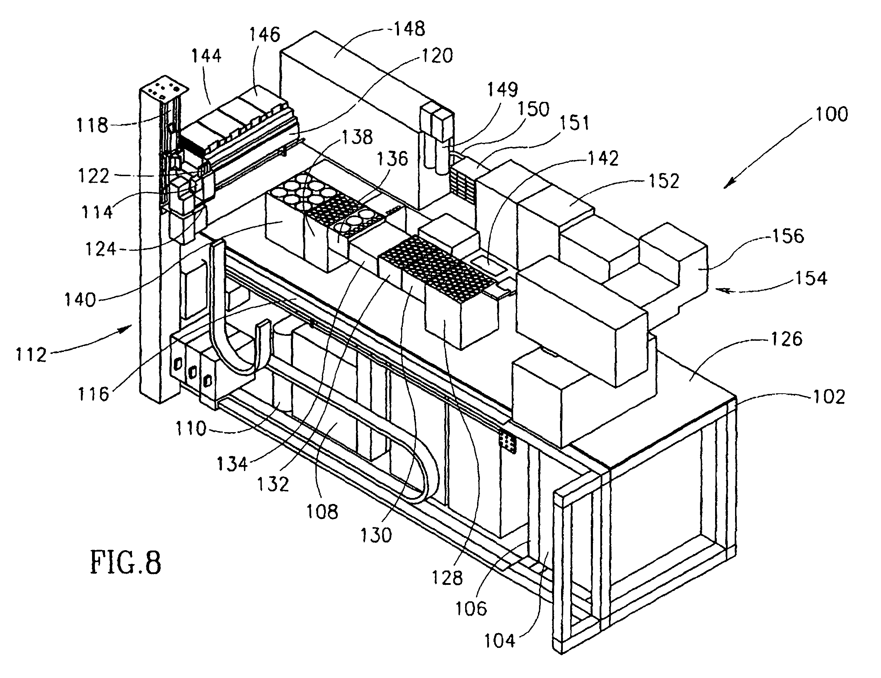

While the method of the invention may be performed manually, this is obviously not practical. In accordance with a preferred embodiment, this is performed in a device which comprises a robotic system for manipulation of the samples, specimens and reagents and comprises a computerized-based, microscope-coupled analysis system. A device useful for the performance of the methods of the invention is shown in Fig. 8. The device generally designated **100** comprises a support frame **102** which houses an electronic control module **104,** a computer **106,** and a vacuum tank **110.** The support frame further supports a four axes robotic system generally designated **112** which comprises a manipulating head **114** which has freedom of movement in the **X, Y** and **Z** directions by means of guiding rails **116, 118** and **120,** respectively. The robotic manipulating head **114** comprises four liquid dispensing end effector units **122** which are adapted to cooperate with disposable microtips for collecting and then dispensing specimens and liquid reagents. In addition, the robotic head also comprises a multi-purpose rotary gripper **124** adapted for gripping sample or specimen containing tubes as well as of multi-compartmental plates.

The support frame supports a table **126** holding three storage boxes **128**, **130** and **132**, each containing disposable tips of different sizes, some serving for the collection and dispensing of specimens and others for collecting and dispensing of reagents. Also held on the table are a box **134** holding a plurality of glass vials for the preparation and holding of diluted urine samples, as well as boxes **136** and **138** containing glass vials containing liquid reagent systems. Held on the table is also a box **140** containing a plurality of glass vials which contain in turn the assayed urine samples. The device further comprises a disposal pit **142** for disposing of used tips, vials, plates, etc.

The device further has a stand **144** holding a plurality of multi-compartmental plates **146,** an incubator **148,** adjusted to about 37°C, a set of medium dispensers **149**, which may be manipulated by the robotic head for dispensing the medium, a stand **150** holding a plurality of filter units **151,** a filtration station **152** for filtering the specimens contained in plates **146** through the filter of filter units **151,** and a microscope unit **154** which comprises a fluorescent lighting system **156** and a specimen viewing stage **158**, having an integral image acquisition device which in this specific embodiment is a video camera connected to the computer which performs the image analysis. Further held on table **126** is a cassette filling station **160.**

In operation, the robotic head **114,** by means of its grippers **124,** extracts one of plates **146** and places it on cassette filling stage **160.** Then, by use of its dispensers **122** the robotic head fills liquid specimens and reagents into the various compartments of plate **146** and following their filling, the plate is typically placed in the incubator **148.** Following incubation, the robotic head **114,** again by means of its grippers **124** extracts the plate **146** from the incubator, places it on one rack **160** and then pulls out a filter unit **151** and places it on top of plate **146**. The gripper which is also able to rotate around a horizontal axis, then grips the assembly comprising the plate and the filter unit, rotates it and places this assembly into the filtration unit for filtration. The spent plate **146** is then dispensed into pit **142** and the filter unit is transported to microscope **154** for viewing. After viewing the filter unit is dispensed as well.

The invention will now be further illustrated by the following specific examples:

### EXAMPLES

### Example 1: Screening of a urine sample for the detection of micro-organisms - enumeration

### Materials:

All fluorescent materials contained in the staining mixtures described below were purchased from Molecular Probes, Inc. (Eugene, Oregon, USA). Glycerol GR (99.5% min. purity) was obtained from Merck. Fluorescent beads (Fluoresbrite plain YG 1.0 μm microspheres) were purchased from Polysciences, inc. (Warrington, PA, USA). Fluorescent beads were prepared at a final concentration of $2.5 \times 10^{-6}$% in 0.02% Na azide, 10% glycerol. Two staining reagent mixtures were prepared, staining reagent mixture I (SRM-1) and staining reagent mixture 2 (SRM-2). SRM-1 is a mixture containing final concentrations of glycerol, SYTO13 and propidium iodide of 34.3%, 14.3μM, and 9 μg/ml, respectively. SRM-2 is a mixture containing final concentrations of glycerol, SYTO13, and hexidium iodide of 34.3%, 9.9 μM, and 7.3 μg/ml, respectively.

### Enumeration Procedure:

A sample of midstream urine was obtained in a cup and shaken for 180 second.

2 ml of the urine sample were collected from the original sample cup and 30 μl duplicate specimens of the urine sample were each deposited into a well of a microtiter culture plate.

To well #1 comprising one specimen of the urine sample, 70 μl of SRM-1 (see above) were added and to well #2 containing the duplicate specimen, 70 μl of SRM-2 (see above) were added. 100 μl of fluorescent beads (prepared as described above) were then deposited into each well and the microplate was shaken for 60 sees.

Following shaking of the plate, the bottom of the culture plate carrying the filter membrane was assembled onto the main body of the culture plate, the plate was inverted and vacuum was applied for 15 mins. during which all the fluid in the culture plate was transferred through the membrane while the stained microorganisms were retained on the membrane surface. The membrane carrying bottom of the culture plate was then subjected to optical analysis in a fluorescent microscope.

Four microscopic fields were analyzed for each location on the filter. The total number of microorganisms as well as the number of dead microorganisms were assessed from the specimen which was incubated with staining reagent system #1. The number of Gram+ and Gram- microorganisms was assessed by analysis of the results of the specimens incubated with the SRM-2 in combination with the results of well #1 (number of dead cells). On the basis of these results, the number of pathogenic microorganisms in the original urine sample was calculated.

The enumeration results of 399 urine specimens which were analyzed as described above, were compared with the results obtained by standard methods which are used routinely at the clinic (as described in Henry D. Isenberg (ed.), Clinical Microbiology Procedures Handbook, ASM Press, 1992). All the urine samples which were compared comprised a threshold of $10^5$ bacteria/ml, and $5 \times 10^5$ polymorphonuclear (PMN) cells/ml. Of the 399 specimens which were analyzed, 22.8% of the specimens were classified as positively infected by pathogenic microorganisms.

The comparison of the results obtained by the two above methods is shown in the following Table 1.

## Table 1

| 2 | TP (%) | TN (%) | FP (%) | FN (%) | sensitivity (%) | specificity (%) | PPV (%) | NPV (%) |
|---|---|---|---|---|---|---|---|---|
| excluding PMN | 18.0 | 69.2 | 8.0 | 4.8 | 79.1 | 89.6 | 69.2 | 93.6 |
| including PMN | 20.6 | 67.7 | 9.5 | 2.3 | 90.1 | 87.7 | 68.3 | 96.8 |

TP = true positive          TN = true negative
FP = false positive         FN = false negative
PPV = negative predictive value = TP/(TP+FP)
NPV = negative predictive value = TN+FN)
sensitivity = TP/(TP+FN)
specificity = TN/(TN+FP)

As seen in the above table. there was a very high correlation between the results obtained by the enumeration analysis method of the present invention and standard analysis method.

### Example 2: Microorganism identification procedure

In the following Example, the following abbreviations will be used:

**TSB** - Tryptic Soy Broth
**EDTA** - Ethylene di-amine tetra-acetate (Sodium salt)
**PBS** - 0.1 M Na-Phosphate buffer pH 7.4, 0.15 M NaCl
**BSA** - Bovine Serum Albumin
**FITC** - Fluorescein-iso-thio-cyanate
**TM -** Perstorp Biotech Company
**PI** - Propidium iodide.

### Identification procedure:

60 μl were collected from each tested urine sample and diluted in TSB growth medium according to the bacterial count determined by the enumeration process (See Example 1) to obtain an initial bacterial concentration of less or equal to $5 \times 10^5$ microorganisms/ml. Each of the diluted urine specimens were dispensed into two wells of microtiter culture plates and the culture plates were then incubated at 37°C for 45 mins.

Following incubation, a permeabilizing agent (Triton X-100 (0.040%), EDTA (20 mM)) was added to all the wells at a volume of 20 μl/well and the microculturc plates were then mixed for 15 sees. at 600 RPM. The microplates were then heated at 82°C for 15 mins. after which they were cooled at room temperature for 5 mins.

The duplicate specimens obtained from each urine sample were divided so that to one specimen in one well, a volume of 20 μl of an anti *E. coli* monoclonal antibody conjugated to biotin (50 μg/ml diluted in PBS and 5% BSA) was added. To the second duplicate specimen in a second well 20 μl of an anti-*Klebsiella spp.* specific monoclonal antibody conjugated to biotin (50 μg/ml diluted in PBS, 5% BSA) was added.

The microculture plates were then mixed again as described above and incubated at 37°C for 30 mins.

After removal of the culture plates from the incubator, 20 µl of a mixture of specific and general stain reagents comprising FITC-Nutravidin™ (50µg/ml in PBS, 5% BSA) and PI (18.75 µg/ml) were added to each of the specimens.

The microplates were then mixed again as described above, incubated at 37°C for 30 mins. after which a volume of 100 µl of 48% glycerol, 0.1% Triton X-100 was added to each well and the microplates were again mixed as described above.

The filter membrane was then assembled onto the microplate which was then inverted and subjected to vacuum for 15 mins. during which the fluid from the microplates were filtered through the poly-carbonate membranes (0.2 µ) while the stained microorganisms were detained on the membrane surface.

The membranes were then transferred to a fluorescent microscope for optical analysis. A positive fluorescent signal in a location on the membrane in which a specimen was incubated with a specific anti *E. coli* monoclonal antibody indicated the presence of *E. coli* bacteria in the original tested urine sample. Similarly, a positive fluorescent signal in a location on the membrane in which a specimen was incubated with a specific anti *Klebsiella spp.* monoclonal antibody indicated the presence of such microorganisms in the original tested urine sample.

On the basis of these results, several characteristics of the above identification process were calculated and are shown in the following Table 2.

Table 2

| MAb specific to | Specificity (%) | Sensitivity (%) | PPV Positive predictive value (%) | NPV Negative predictive value (%) |
|---|---|---|---|---|
| *E. coli* | 95.5 | 96.2 | 93.9 | 97.7 |
| *Klebsiella spp.* | 93.6 | 88.8 | 66.6 | 98.2 |
| % Specificity = TN/(TN+FP)<br>% Sensitivity = TP/(TP+FN) | | PPV = (TP+FP)<br>NPV = TN+FN | | |

As seen in the above table, the results obtained by the above method were highly correlated with results obtained for the same samples by standard methods.

**Example 3: Sensitivity of the identified microorganisms in a sample to anti-microbial agents**

<u>Materials:</u>

All reagents used in the assay described below were filter sterilized using 0.2 µm membrane filters.

**Growth medium**

MHX2 Times 2 final recommended concentration of cation adjusted Mueller Hinton broth (BBL) prepared according to the manufacturers' instructions.

Tryptic Soy Broth (TSB) as described in Example 2 above.

**Anti-microbial agents**

Working stock solutions were prepared by solubilizing in water according to the manufacturers' instructions. Working stock concentrations and final concentrations (in µg/ml) were as follows:

| Anti-microbial agent | Working Stock (µg/ml) | Final Low Conc. (µ/g/ml) | Final High Conc. (µg/ml) |
|---|---|---|---|
| Amikacin | 80 | 8 | 16 |
| Ampicillin - Gram⁻ & Enterococci | 20 | 2 | 4 |
| Ampicillin - Staphylococcus | 1.25 | 0.125 | 0.25 |
| Ampicillin - Streptococcus | 2.5 | 0.25 | 0.5 |
| Amoxicillin/clavulanate | 40 | 4 | 8 |
| Cefazolin | 20 | 2 | 4 |

(continued)

| Anti-microbial agent | Working Stock (μg/ml) | Final Low Conc. (μ/g/ml) | Final High Conc. (μg/ml) |
|---|---|---|---|
| Ciprofloxacin | 5 | 0.5 | 1 |
| Cefotaxime | 20 | 2 | 4 |
| Ceftazidime | 20 | 2 | 4 |
| Gentamicin | 40 | 4 | 8 |
| Oxacillin | 5 | 0.5 | 1 |
| Piperacillin | 20 | 2 | 4 |
| Trimethoprim/ sulfamethaxozole | 10 | 1 | 2 |
| Vancomycin | 10 | 1 | 2 |

**Fluorescent stain**

Non specific stain (NSS) working stock contained (in water): Glycerol 40% (v/v); Propidium Iodide 12.5 μg/ml; Triton X 100 0.01%; 10 mM EDTA-NaCl.

**Distilled water**

Autoclave sterilized, double distilled water (DDW) was used.

**Sensitivity procedure:**

Samples of urine from patients suffering from urinary tract infections were diluted to a starting concentration of $5 \times 10^5$ cells/ml in sterile water. An appropriate volume of each urine sample was then mixed with MHX2 growth medium at a ratio of 3/5 and 80 μl of the resulting suspension was dispensed into each separate well of microculture plates. Each sample was divided into four such above specimens. Two specimens of each sample served as control specimens to which no anti-microbial agent was added. To the other two specimens of each sample a tested anti-microbial agent was added at a low concentration to one well and at a high concentration to a second well. The contents of each well are shown in the following Table 3.

Table 3

| Assay component | Anti-microbial agent (μl) | DDW (μl) |
|---|---|---|
| Growth controls | - | 20 |
| Low anti-microbial agent concentrations | 10 | 10 |
| High anti-microbial agent concentrations | 20 | - |

The identity of the microorganisms present in each of the urine samples was assessed by the identification process generally described in the specification above and specifically in Example 2 above. Based on the identity results obtained by the above method, several kinds of anti-microbial agents were added to each sample as described in the following Table 4.

Table 4

| Anti-microbial agent | Unknown[1] | Gram-[2] | Gram+[3] |
|---|---|---|---|
| Amikacin | + | + | + |

[1] tested against microorganisms which gram staining is unknown

[2] tested against Gram⁻ microorganisms

[3] tested against Gram⁺ microorganisms

Table 4   (continued)

| Anti-microbial agent | Unknown[1] | Gram⁻[2] | Gram⁺[3] |
|---|---|---|---|
| Ampicillin | + | + | + |
| Amoxicillin/clavulanate | + | + | + |
| Cefazolin | + | + | + |
| Ciprofloxacin | + | + | + |
| Cefotaxime | + | + | - |
| Ceftazidime | + | + | - |
| Gentamicin | + | + | + |
| Oxacillin | + | - | + |
| Piperacillin | + | - | + |
| Trimethoprim/sulfamcthaxozole | + | + | + |
| Vancomycin | + | - | - |

[1] tested against microorganisms which gram staining is unknown

[2] tested against Gram⁻ microorganisms

[3] tested against Gram⁺ microorganisms

The microculture plates were then centrifuged at 600 rpm for 1 min. after which they were incubated at 37°C for 2 hours.

Following the incubation, 100 µl of the staining reagent - NSS was added to each well and the microplates were centrifuged again at 600 rpm for 1 min. and incubated at 82°C for 12 mins.

Following the last incubation, the fluid contents of the microplates was filtered onto an 0.2 µm membrane filter by applying a vacuum for 15 mins. as described in Examples 1 and 2 above wherein the fluid was filtered through the filter and the stained microorganisms were detained on the membrane surface.

The membrane comprising the stained microorganisms was then subjected to analysis under a fluorescent microscope. Four different microscope fields of each location on the membrane were analyzed and the sensitivity of each of the microorganisms in the urine samples to each anti-microbial agent was determined. The microorganisms were characterized as sensitive, intermediate or resistant to each of the tested anti-microbial agents.

The characterization took into account both the number of surviving microorganisms after their incubation with the anti-microbial drug as well as the change in various morphological parameters of the microorganisms.

The results obtained by the above method were compared to results obtained for the same samples by standard microdilution susceptibility tests (carried out according to the NCCLS guidelines). The % of results which were in total agreement between the two above methods were between about 80% to about 90% and the % of the results which fall into the definition of very major errors was between about 2% to about 6%.

**Claims**

1.   A method for identifying the type of microorganisms contained in a liquid sample, comprising:

(a) preparing a plurality of specimens from the sample;
(b) obtaining a plurality of microorganism-specific staining agents and adding each such agent to at least one specimen, and then incubating for a time sufficient to allow incorporation or binding of the microorganism specific agent to the microorganisms;
(c) filtering the specimens through a filter having pores of a size such that said microorganisms are retained on said filter;
(d) transferring the filter to a stage of a microscope for viewing by the microscope; and
(e) detecting stained microorganisms retained on the filter, the presence of a stained microorganism indicating the presence of a specific microorganism to which the stain is specific in the sample.

2.   A method according to Claim 1, wherein the microorganism-specific staining agents comprise monoclonal antibodies.

17

3. A method according to Claim 2, wherein the monoclonal antibodies are conjugated each to one member of a binding couple capable of specifically binding to another member of a couple and prior to filtering the specimens, a staining complex is added comprising the other member of a binding couple conjugated to a staining agent.

4. A method according to Claim 3, wherein the monoclonal antibodies are conjugated to biotin, and the straining complex comprises avidin conjugated to a fluorescent marker.

5. A method for evaluating the sensitivity of microorganisms in a liquid sample to an anti-microbial agent, the method comprising:

(a) obtaining data on type of microorganisms in the sample by a method according to any of claims 1 to 4.
(b) preparing a plurality of liquid specimens from the sample;
(c) adding the anti-microbial agent to at least one specimen and incubating for a time sufficient for the anti-microbial agent to exert an effect on microorganisms sensitive thereto; at least one other specimen serving as control and being incubated under identical conditions without adding an anti-microbial agent;
(d) adding a stain based on said data such that where said data indicates the presence in the sample of a single population of microorganisms, adding a general microorganism stain, and where said data indicates the presence of more than one population of microorganisms, adding a general strain, and adding a specific stain for each type of microorganisms in the samples, save for one;
(e) filtering the specimens through a filter having pores such that said microorganisms are retained on said filter;
(f) transferring the filter with the retained microorganisms to a stage of a microscope for viewing by the microscope; and
(g) detecting microorganisms on the filter and evaluating their sensitivity to the anti-microbial agent based on a change in one or more properties of the microorganisms, comprising:

(ga) the number of live microorganisms
(gb) morphological parameters of the microorganisms; and
(gc) biochemical properties manifested by color or color patter in the microorganisms.

6. A method according to claim 5, comprising incubating a plurality of specimens, at least one of which serving as control and the others are being incubated each with a different anti-microbial agent from a panel of anti-microbial agents, each one of the microbial agents from the panel being added to at least one specimen.

7. A method according to Claim 5 or 6, wherein the anti-microbial agents which are tested are selected based on data relating to the identity of microorganisms in the sample.

8. A method according to any of Claims 1 to 7, wherein the liquid sample is a body fluid.

9. A method according to Claim 8, wherein the body fluid is urine.

10. A system **(100)** for carrying out a microbiological assay, the system **(100)** comprising:

(a) a sample placing station **(140)** for introduction of samples to be assayed;
(b) one or more devices **(136, 138)** comprising or capable of holding a plurality of liquid reagent containing vessels;
(c) a device **(144)** for holding a plurality of specimen plates **(146),** each comprising a plurality of specimen holding compartments, and a device **(150)** for holding a plurality of filter units **(151),** each comprising a stretched filter sheet;
(d) a filtration unit **(152)** comprising a vacuum stage for filtering specimens contained in the specimen plate **(146)** through the filter sheet of the filter unit **(151);**
(e) an incubator unit **(148)** adapted for holding a plurality of specimen plates **(146);**
(f) a microscope unit **(154)** comprising a microscope, a stage adapted for holding a filter unit for viewing of particulate matter contained on the filter sheet by the microscope, the microscope having an integral image acquisition device connected to a computerized image analysis system; and
(g) a robotic manipulating unit **(112),** having grippers **(122)** for holding and manipulating sample containing vessels, specimen holding plates, and filtering units, and having dispenser devices for collecting liquid samples, specimens and reagents and discharging them into compartments of the specimen plates.

**EP 0 944 733 B1**

**Patentansprüche**

1. Verfahren zur Identifizierung des Typs eines in einer flüssigen Probe enthaltenen Mikroorganismus, umfassend:

    a) die Bereitung mehrerer Examplare der Probe;
    b) die Beschaffung mehrerer für Mikroorganismen spezifischer Färbemittel und Zusatz jedes dieser Mittel zu mindestens einem Probenexemplar und anschließend ausreichend langes Inkubieren, um die Einführung des für den Mikroorganismus spezifischen Mittels in die Mikroorganismen oder das Binden an die Mikroorganismen zu ermöglichen;
    c) das Filtrieren der Probenexemplare durch ein Filter mit Poren in einer derartigen Größe, dass die Mikroorganismen auf dem Filter zurückgehalten werden;
    d) das Aufbringen des Filters auf die Bühne eines Mikroskops zur Betrachtung durch das Mikroskop; und
    e) das Feststellen der auf dem Filter zurückgehaltenen Mikroorganismen, wobei die Anwesenheit eines gefärbten Mikroorganismus die Anwesenheit eines speziellen Mikroorganismus für den die Färbung spezifisch ist, in der Probe anzeigt.

2. Verfahren nach Anspruch 1, bei dem die für Mikroorganismen spezifischen Färbemittel monoklonale Antikörper umfassen.

3. Verfahren nach Anspruch 2, bei dem die monoklonalen Antikörper jeweils an einen Teil eines Bindungspaares konjugiert sind, das zur spezifischen Bindung an einen anderen Teil eines Paares geeignet ist, und vor dem Filtrieren der Probenexemplare ein färbender Komplex zugefügt wird, der den anderen Teil eines Bindungspaares, konjugiert an ein Färbemittel, umfasst.

4. , Verfahren nach Anspruch 3, bei dem die monoklonalen Antikörper an Biotin konjuglert sind und der färbende Komplex Avidin, konjugiert an einen fluoreszierenden Marker, umfasst.

5. Verfahren zur Bewertung der Empfindlichkeit eines Mikroorganismus in einer flüssigen Probe gegen ein antimikrobielles Mittel, umfassend:

    a) Erstellen der Daten des Typs des Mikroorganismus in der Probe nach dem Verfahren eines der Ansprüche 1 bis 4,
    b) Herstellen mehrerer flüssiger Exemplare der Probe
    c) Zusatz des antimikrobiellen Mittels zu mindestens einem Probenexemplar und Inkubieren während einer Zeit die ausreicht, dass das antimikrobielle Mittel auf den dafür empfindlichen Mikroorganismus einwirkt, wobei mindestens ein anderes Probenexemplar, das als Kontrolle dient, unter identischen Bedingungen ohne Zusatz eines antimikrobiellen Mittels inkubiert wird;
    d) Zusatz eines Färbemittels, basierend auf diesen Daten, derart dass wenn diese Daten die Anwesenheit einer einzigen Mikroorganismenpopulation In der Probe anzeigen, ein allgemeiner Organismenstamm zugesetzt wird und wenn diese Daten die Anwesenheit von mehr als einer Mikroorganismenpopulation anzeigen, ein allgemeiner Stamm und ein spezifischer Stamm für jeden Mikroorganismentyp in den Proben, mit Ausnahme von einer, zugefügt wird;
    e) Filtrieren der Proben durch ein Filter mit Poren, die die Mikroorganismen auf dem Filter zurückhalten;
    f) Überführen des Filters mit den zurückgehaltenen Organismen auf die Bühne eines Mikroskops zur Betrachtung durch das Mikroskop; und
    g) Feststellen der Mikroorganismen auf dem Filter und Bewertung ihrer Empfindlichkeit für das antimikrobielle Mittel basierend auf einer Änderung von einer oder mehreren Eigenschaften der Mikroorganismen, umfassend:

        (ga) die Anzahl der lebenden Mikroorganismen
        (gb) die morphologischen Parameter der Mikroorganismen; und
        (bc) die biochemischen Eigenschaften, die sich durch die Farbe oder das Farbmuster In den Mikroorganismen manifestieren.

6. Verfahren nach Anspruch 5, umfassend das Inkubieren mehrerer Probeexemplare, wobei mindestens eines davon als Kontrolle dient und die anderen jeweils mit einem unterschiedlichen antimikrobiellen Mittel aus einer Reihe von antimikrobiellen Mittein inkubiert werden, wobei jedes der mikrobiellen Mittel aus der Reihe zu mindestens einem Probenexemplar gefügt wird.

19

7. Verfahren nach Anspruch 5 oder 6, bei dem die untersuchten antimikrobiellen Mittel ausgewählt werden auf der Basis von Daten, die von der Identität des Mikroorganismus in der Probe abhängen.

8. Verfahren nach einem der Ansprüche 1 bis 7, bei dem die flüssige Probe eine Körperflüssigkeit ist.

9. Verfahren nach Anspruch 8, bei dem die Körperflüssigkeit Urin ist.

10. System (100) zur Durchführung eines mikrobiologischen Essays, wobei das System (100) umfasst:

a) eine Einrichtung zur Aufnahme von Proben (140) zur Einführung der zu untersuchenden Proben;
b) eine oder mehrere Einrichtungen (136, 138), die mehrere flüssiges Reagens enthaltende Gefäße umfassen oder zur deren Halterung geeignet sind;
c) eine Vorrichtung (144) zur Halterung mehrerer Probenexemplar-Platten (146), wobei jede mehrere Abschnitte zur Halterung von Probenexemplaren aufweist, sowie eine Vorrichtung (150) zur Halterung mehrerer Filtereinheiten (151), wovon jede einen gestreckten Filterbogen umfasst;
d) eine Filtriereinheit (152), umfassend eine Vakuumstufe zur Filtration von Probenexemplaren, die in der Probenexemplarplatte (146) enthalten sind, durch den Filterbogen der Filtereinheit (151);
e) eine Inkubatoreinheit (148), geeignet zur Halterung mehrerer Probenexemplar-Platten (146);
f) eine Mikroskopeinheit (154), umfassend ein Mikroskop, eine Bühne, geeignet zur Halterung einer Filtereinheit zur Betrachtung von teilchenförmigem Material, das auf dem Filterbogen enthalten ist, durch das Mikroskop, wobei das Mikroskop eine integrale Bildaufnahmeeinrichtung aufweist, die mit einem computerisierten Bildanalysesystem verbunden ist; und
g) eine Roboter-Manipulationseinheit (112) mit Greifern (122) zur Halterung und Manipulation von Proben enthaltenden Gefäßen, Probenexemplare halternden Platten und Fittereinheiten und mit Verteilereinrichtungen zur Aufnahme flüssiger Proben, Probenexemplare und Reagentien, und deren Verteilung in Abschnitten der Probenexemplar-Platten.

## Revendications

1. Procédé pour l'identification du type de micro-organismes contenus dans un échantillon liquide, comprenant :

(a) la préparation d'une pluralité de prises d'essai à partir de l'échantillon ;
(b) l'obtention d'une pluralité d'agents de coloration spécifiques de micro-organismes et l'addition de chacun de ces agents à au moins un spécimen, et ensuite l'incubation pendant une durée suffisante pour permettre l'incorporation ou la fixation aux micro-organismes de l'agent spécifique de micro-organisme ;
(c) la filtration des prises d'essai à travers un filtre ayant des pores d'une taille telle que lesdits micro-organismes sont retenus sur ledit filtre ;
(d) le transfert du filtre sur la platine d'un microscope pour l'examen au microscope ; et
(e) la détection des micro-organismes colorés retenus sur le filtre, la présence d'un micro-organisme coloré indiquant la présence d'un micro-organisme spécifique pour lequel la coloration est spécifique dans l'échantillon.

2. Procédé selon la revendication 1, dans lequel les agents de coloration spécifiques de micro-organismes comprennent des anticorps monoclonaux.

3. Procédé selon la revendication 2, dans lequel les anticorps monoclonaux sont conjugués chacun à un membre d'un couple de liaison capable de se lier spécifiquement à un autre membre d'un couple et, avant la filtration des spécimens, on ajoute un complexe colorant comprenant k'autre membre d'un couple de liaison conjugué à un agent de coloration.

4. Procédé selon la revendication 3, dans lequel les anticorps monoclonaux sont conjugués à la biotine, et le complexe colorant comprend de l'avidine conjuguée à un marqueur fluorescent.

5. Procédé pour l'évaluation de la sensibilité de micro-organismes dans un échantillon liquide à un agent antimicrobien, le procédé comprenant

(a) l'obtention de données sur le type de micro-organismes dans l'échantillon par un procédé selon l'une quelconque des revendications 1 à '4;

(b) la préparation d'une pluralité de prises d'essai liquides à partir de l'échantillon ;

(c) l'addition de l'agent antimicrobien à au moins une prise d'essai et l'incubation pendant une durée suffisante pour permettre à l'agent antimicrobien d'exercer un effet sur les micro-organismes sensibles à celui-ci ; au moins une autre prise d'essai servant de témoin et étant mise à incuber dans des conditions identiques sans addition d'un agent antimicrobien;

(d) l'addition d'un colorant basé sur lesdites données, telle que lorsque lesdites données indiquent la présence dans l'échantillon d'une seule population de micro-organismes, l'addition d'un colorant pour micro-organismes généraux, et lorsque lesdites données indiquent la présence de plus d'une population de micro-organismes, l'addition d'un colorant général, et l'addition d'un colorant spécifique pour chaque type de micro-organismes dans les échantillons, hormis un seul ;

(e) la filtration des prises d'essai à travers un filtre ayant des pores d'une taille telle que lesdits micro-organismes sont retenus sur ledit filtre ;

(f) le transfert du filtre avec les micro-organismes retenus sur la platine d'un microscope pour l'examen au microscope ; et

(g) la détection des micro-organismes sur le filtre et l'évaluation de leur sensibilité audit agent antimicrobien, sur la base d'un changement d'une ou plusieurs propriétés des micro-organismes, comprenant :

   (ga) le nombre de micro-organismes vivants
   (gb) les paramètres morphologiques des micro-organismes ; et
   (gc) les propriétés biochimiques manifestées par la couleur ou le profil de coloration dans les micro-organismes.

6. Procédé selon la revendication 5, comprenant l'incubation d'une pluralité de spécimens, au moins l'un d'eux servant de témoin et les autres étant mis à incuber chacun avec un agent antimicrobien différent parmi une galerie d'agents antimicrobiens, chacun des agents antimicrobiens de la galerie étant ajouté à au moins un spécimen.

7. Procédé selon la revendication 5 ou 6, dans lequel les agents antimicrobiens qui sont testés sont choisis sur la base de données relatives à l'identité des micro-organismes dans l'échantillon.

8. Procédé selon l'une quelconque des revendications 1 à 7, dans lequel l'échantillon liquide est un liquide corporel.

9. Procédé selon la revendication 8, dans lequel le liquide corporel est l'urine.

10. Système (100) pour l'exécution d'un dosage microbiologique, le système (100) comprenant :

   (a) un poste (140) de dépôt d'échantillons pour l'introduction d'échantillon à tester ;
   (b) un ou plusieurs dispositifs (136, 138) comprenant ou pouvant maintenir une pluralité de récipients contenant des réactifs liquides ;
   (c) un dispositif (144) pour supporter une pluralité de plaques (146) de spécimens, comprenant chacune une pluralité de compartiments de support de spécimens, et un dispositif (150) pour supporter une pluralité d'unités de filtration (151), comprenant chacune une feuille filtrante tendue ;
   (d) une unité de filtration (152) comprenant un étage de mise sous vide pour la filtration des prises d'essai contenues dans la plaque de prises d'essai (146) à travers la feuille filtrante de l'unité de filtration (151) ;
   (e) une unité d'incubation (148) destinée à supporter une pluralité de plaques de prises d'essai (146) ;
   (f) une unité de microscope (154) comprenant un microscope, une platine destinée à supporter une unité de filtration pour l'examen au microscope de matière particulaire contenue sur la feuille filtrante, le microscope comportant un dispositif d'acquisition d'image relié à un système d'analyse d'image par ordinateur ; et
   (g) une unité de manipulation robotique (112), comportant des pinces (122) pour le maintien et la manipulation de récipients contenant des échantillons, des plaques supportant des spécimens, et des unités de filtration, et comportant des dispositifs distributeurs pour la collecte d'échantillons liquides, de prises d'essai et de réactifs et leur décharge dans les compartiments des plaques de spécimens.

FIG.1

FIG.2

EP 0 944 733 B1

FIG.3A

FIG.3B

FIG.3B (CONT.)

```
TOTAL : 50
DEAD  :  6 (2 RODS
                4 COCCI)
―――――――――――――――――
LIVE  : 44

  ●        LIVE  COCCUS
  ○        DEAD  COCCUS
 ═══       LIVE  ROD
 ━━━       DEAD  ROD
```

FIG.4A

```
TOTAL : 50
GRAM⁺ :  8 (RODS)
―――――――――――――――――
TOTAL : 42
(EXCLUDING G⁺RODS)

DEAD COCCI :  4
―――――――――――――――――
PATHOGENIC : 38

  ●        COCCUS  GRAM⁻
  ○        COCCUS  GRAM⁺
 ═══       ROD  GRAM⁻
 ━━━       ROD  GRAM⁺
```

FIG.4B

FIG.5A

FIG.5B

FIG.5C

FIG.5D

70

DILUTION

71

84

82

ADD M.O SPECIFIC Abs (Abs CONJUGATED TO BIOTIN)

$Ab_1$     $Ab_2$     $Ab_3$     $Ab_{n-1}$     $Ab_n$

ADD AVIDIN – FITC

FILTERATION

MICROSCOPIC EVALUATION

+     –     –     +     –

RESULTS : 2 M.O.s IN SAMPLE : $M.O._1$ AND $M.O._{n-1}$

FIG.6

FIG.7

FIG.7 (CONT.)

MICROSCOPIC | ANALYSIS

NUMBER          MORPHOLOGY

SENSITIVITY

FIG.8

EP 0 944 733 B1